(19) Europäisches Patentamt
European Patent Office
Office européen des brevets

(11) **EP 4 220 166 A1**

(12) **EUROPEAN PATENT APPLICATION**

(43) Date of publication:
**02.08.2023 Bulletin 2023/31**

(21) Application number: **22382087.9**

(22) Date of filing: **01.02.2022**

(51) International Patent Classification (IPC):
*G01N 33/543* (2006.01)   *G01N 33/558* (2006.01)
*G01N 33/569* (2006.01)   *G01N 33/58* (2006.01)
*G01N 33/68* (2006.01)

(52) Cooperative Patent Classification (CPC):
**G01N 33/54306; G01N 33/558; G01N 33/569;
G01N 33/56983; G01N 33/587; G01N 33/6854**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Universitat Rovira I Virgili (URV)
43003 Tarragona (ES)**
• **Institució Catalana De Recerca I
Estudis Avançats (ICREA)
08010 Barcelona (ES)**

(72) Inventors:
• **O´SULLIVAN, Ciara
43007 Tarragona (ES)**
• **JAUSET RUBIO, Miriam
43007 Tarragona (ES)**
• **SKOURIDOU, Vasoula
43007 Tarragona (ES)**

(74) Representative: **Hoffmann Eitle
Hoffmann Eitle S.L.U.
Paseo de la Castellana 140, 3a planta
Edificio LIMA
28046 Madrid (ES)**

(54) **IN VITRO METHOD FOR DETECTING ANTIBODIES IN A SAMPLE**

(57)    The present invention relates to an *in vitro* method for detecting one or more antibodies in a sample using a first detector comprising an antibody binding molecule conjugated to a first detectable entity that comprises carbon nanoparticles and, preferably, an enzyme and a capture entity capable of specifically binding to the antibody and being immobilized on a test region of a surface. The present invention furthermore relates to devices for performing said methods.

EP 4 220 166 A1

**Description**

<u>TECHNICAL FIELD</u>

The present invention relates to an *in vitro* method for detecting one or more antibodies in a sample using a first detector comprising an antibody binding molecule conjugated to a first detectable entity that comprises carbon nanoparticles and, preferably, an enzyme and a capture entity capable of specifically binding to the antibody and being immobilized on a test region of a surface. The present invention furthermore relates to devices for performing said methods.

<u>BACKGROUND ART</u>

There is an increasing demand for convenient and accurate point-of-care tools that can detect and diagnose diseases and ideally detect the different stages of the disease, even in remote or economically less affluent settings. In recent years, lateral flow Immunoassays (LFIA) have been indicated as a suitable medical diagnostic tool for these environments because they require little or no sample preparation, provide rapid and reliable results with no electronic components and thus can be manufactured at low costs and be operated by unskilled personnel. Lateral Flow Immunoassays are immunoassays that can be used to detect biological agents including analytes and also antigens. They are based on the recognition of one or more analyte of interest by using antibodies. The antibodies are fixed onto a nitrocellulose membrane and they interact with the analyte either in sandwich or competitive formats using a proper label. The main advantage compared to other immunosensors is that the entire assay can be done in one step and in a few minutes. However, the limit of detection is generally not as good as in other assays like ELISA. Even though they have been successfully applied to acute and chronic disease detection, LFIA can still show serious limitations when high sensitivity is needed.

(COVID-19) is a viral infectious respiratory disease first reported at the end of 2019 in the Hubei province of China causing atypical pneumonia. The new coronavirus was then named as SARS-CoV-2. Like other coronaviruses of the same family, SARS-CoV-2 is an enveloped beta coronavirus with a virion of 50 - 200 nm diameter containing a positive-sense single-stranded RNA viral genome of approximately 30,000 bp. It has four structural proteins: spike (S), membrane (M), envelope (E) and nucleoprotein (N). S, M and E proteins create the viral envelope which contains the viral RNA genome bound to multiple copies of the N protein. The S protein is also responsible for receptor-binding enabling the virus to enter the cell and replicate.

The availability of multiple viral genome sequences at the initial stages of the outbreak facilitated the rapid development of nucleic acid amplification tests (NAAT) which were employed for the diagnosis of SARS-CoV-2 infections. Specifically, reverse transcriptase real time PCR (RT-PCR) using virus-specific primers was designated as the reference detection method and several laboratory RT-PCR kits were developed as a result. RT-PCR molecular testing, however, is associated with some inherent limitations related to turn-around times, reagents and instruments costs, personnel training as well as false negative results observed specifically during SARS-CoV-2 testing. More importantly, RT-PCR is not compatible with rapid and easy point-of-care patient testing. It is therefore evident that alternative rapid, sensitive and specific tests suitable for on-site testing are needed to implement the virus containment efforts.

Serological antibody tests, such as ELISA or LFIA, can serve as these alternatives and can complement NAAT testing, as well as being used for detection of individuals who previously had the infection but were not aware of it. They rely on the detection of the IgM, IgA and IgG antibodies specifically produced by the human body's immune system to bind specific regions of the virus's structural proteins (S, E, M and N) as a response to the viral infection. IgM and IgA antibodies are the first isotype to be produced soon after infection, whereas isotype IgG antibodies are produced next and are related to long term immunity and immunological memory. The presence of these antibodies in a patient's blood can therefore provide information on both current and past infections and their detection can efficiently complement the containment efforts and determine the true extent of the infection considering also the plethora of asymptomatic COVID-19 patients.

An extensive list of currently commercially available and under development serological test kits including ELISA and lateral flow assays can be found [https://www.finddx.org/covid-19/pipeline/]. As described above LFIAs are more relevant, since they can be easily and quickly performed by anyone requiring no additional material, lab infrastructure or specialized training. Two main formats have been developed, but both suffer some serious drawbacks. In one format, protein or peptides are immobilised at the test line and detect a combination of IgG and IgM, but cannot differentiate between them, making it impossible to diagnose whether the infection is active or past. The other format used relies on capturing the IgG/IgM human antibodies contained in the specimen using anti-human-IgG/anti-human-IgM antibodies immobilized on the test line and a viral antigen-gold nanoparticle conjugate is used for detection. The viral antigen can be a protein domain, e.g. recombinant receptor binding domain of the S protein or even peptides from any of the structural proteins of the virus. This format however can lead to false negative results since the binding capacity of the test lines will easily be saturated by any IgG/IgM present in the patient's blood stream, which in normal blood are usually at concentrations of 7.5 - 22 mg/mL for IgG and 0.2 - 2.8 mg/mL for IgM. This saturation of the test lines by non-specific IgG and IgM will result in high numbers of false negatives and is a very serious drawback of these tests. IgA antibodies are to date not commonly used in such tests.

In summary, the currently available LFIAs suffer from major drawbacks, such as low sensitivity, unreliable distinction of the presence of IgM, IgA and IgG antibodies in the sample and a high amount of false negative results.

The present invention addresses the above drawbacks provided by the methods of the prior art and provides for a very simple, easy to perform and fast method for detecting the presence of one or more antibodies in a sample, preferably for the detection of COVID-19 IgG and/or IgM and/or IgA antibodies.

## SUMMARY

In one embodiment the present invention therefore provides for an *in vitro* method for detecting an antibody in a test sample comprising the steps of

(a) contacting the test sample with a first detector to form a complex comprising the first detector and the antibody, wherein the first detector comprises an antibody binding molecule conjugated to a first detectable entity, and wherein the first detectable entity comprises carbon nanoparticles and, preferably an enzyme;
(b) contacting the first complex with a capture entity immobilized on a test region of a surface, wherein the capture entity is capable of specifically binding to the antibody; and
(c) detecting the presence of a signal from the first detectable entity in the test region, wherein the presence of the signal is indicative of the presence of the antibody in the test sample.

In a further embodiment the invention provides for an *in vitro* method for detecting an antibody in a test sample comprising the steps of

(a) contacting the test sample with a capture entity immobilized on a test region of a surface, wherein the capture entity is capable of specifically binding to the antibody to form a complex comprising the capture entity and the antibody;
(b) contacting the complex comprising the capture entity and the antibody with a first detector to form a complex comprising the first detector and the antibody, wherein the first detector comprises an antibody binding molecule conjugated to a first detectable entity, and wherein the first detectable entity comprises at least carbon nanoparticles, and preferably an enzyme; and
(c) detecting the presence of a signal from the first detectable entity in the test region, wherein the presence of the signal is indicative of the presence of the antibody in the test sample.

In a preferred embodiment of the present invention the antibody is selected from IgM, IgG, IgD, IgA and IgE, or a combination thereof, preferably wherein the antibody is selected from IgM, IgG and IgA, or a combination thereof.

In a preferred embodiment of the present invention the enzyme used in the methods as described above is selected from horseradish peroxidase, alkaline phosphatase and glucose oxidase, preferably the enzyme used is horseradish peroxidase.

In a further embodiment the first detector is immobilized to a conjugate region of the surface and wherein the conjugate region does not overlap with the test region of the surface.

In one embodiment of the method of the present invention the antibody binding molecule is an anti-IgM, anti-IgG, anti-IgD, anti-IgA or anti-IgE, or a combination thereof, preferably an anti-IgM, anti-IgG or anti-IgA, or a combination thereof.

In a further embodiment the capture entity is an antigen or an antigenic peptide, preferably a SARS-CoV-2 antigen or antigenic peptide.

In one embodiment of present invention said method comprises the simultaneous detection of IgM and IgG or IgA and IgG antibodies in the test sample.

In one preferred embodiment said method comprises the steps of

(a) contacting the test sample with a first detector to form a complex comprising the first detector and the antibody, wherein the first detector comprises an anti-IgM or anti-IgA antibody binding molecule conjugated to carbon nanoparticles and an anti-IgG antibody binding molecule conjugated to a metallic nanoparticle or metallic nanoshell, or wherein the first detector comprises an anti-IgG antibody binding molecule conjugated to carbon nanoparticles and an anti-IgM or anti-IgA antibody binding molecule conjugated to a metallic nanoparticle or metallic nanoshell;
(b) contacting the complex with a capture entity immobilized on a test region of a surface, wherein the capture entity is capable of specifically binding to the antibody; and

(c) detecting the presence of a signal from the combined signal generation after chromogenic substrate addition in the test region, wherein the presence of the signal is indicative of the presence of the antibody in the test sample and of the type of IgM, IgA and/or IgG antibody(ies) present in the test sample.

In one embodiment of this preferred embodiment the metallic nanoparticle or metallic nanoshell is selected from the group consisting of gold nanoparticles, silver particles, copper particles, platinum particles, cadmium particles, composite particles, gold hollow spheres, gold-coated silica nanoshells, and silica-coated gold shells. Preferred are gold nanoparticles.

In a further preferred embodiment said method comprises the steps of

(a) contacting the test sample with a first detector to form a complex comprising the first detector and the antibody, wherein the first detector comprises an anti-IgG antibody binding molecule conjugated to gold nanoparticles and an anti-IgM or anti-IgA antibody binding molecule conjugated to carbon nanoparticles and preferably an enzyme, more preferably horseradish peroxidase, or wherein the first detector comprises an anti-IgM or anti-IgA antibody binding molecule conjugated to gold nanoparticles and an anti-IgG antibody binding molecule conjugated to carbon nanoparticles and preferably an enzyme, more preferably horseradish peroxidase;
(b) contacting the complex with a capture entity immobilized on a test region of a surface, wherein the capture entity is capable of specifically binding to the antibody; and
(c) detecting the presence of a signal from the combined signal generation after chromogenic substrate addition in the test region, wherein the presence of the signal is indicative of the presence of the antibody in the test sample and of the IgM, IgA and/or IgG antibody(ies) present in the test sample.

In one embodiment of the methods of present invention the said conjugate region further comprises a control detector.

In a further embodiment of present invention, the surface is a flow path in a lateral flow assay device.

In one embodiment of the present invention the test sample is a bodily fluid, extract of a bodily organ, blood, serum, or plasma.

The present invention furthermore relates to an antibody detection device comprising:

a sample loading region;
a conjugate region, wherein said conjugate region comprises a mobilizable first detector including an antibody binding molecule conjugated to a first detectable entity, wherein the first detectable entity comprises carbon nanoparticles, and preferably an enzyme; and
a test region, wherein said test region comprises an immobilized capture entity capable of specifically binding to the antibody;
wherein the sample loading region, the conjugate region and the test region are configured so that in operation a liquid sample when loaded into the sample loading region, is in fluid communication with the conjugate region and the test region.

In a preferred embodiment of the detection device the antibody binding molecule is an anti-IgM, anti-IgG, anti-IgD, anti-IgA or anti-IgE, or a combination thereof. Most preferred are anti-IgM, anti-IgG or anti-IgA, or a combination thereof.

In a further embodiment the capture entity is an antigen or antigenic peptide, preferably a SARS-CoV-2 antigen or antigenic peptide.

In one embodiment of the detection device of present invention the first detector comprises an anti-IgM or anti-IgA antibody binding molecule conjugated to carbon nanoparticles and an anti-IgG antibody binding molecule conjugated to a metallic nanoparticle or metallic nanoshell, or the first detector comprises an anti-IgG antibody binding molecule conjugated to carbon nanoparticles and an anti-IgM or IgA antibody binding molecule conjugated to a metallic nanoparticle or metallic nanoshell.

In a preferred embodiment the metallic nanoparticle or metallic nanoshell is selected from the group consisting of gold nanoparticles, silver particles, copper particles, platinum particles, cadmium particles, composite particles, gold hollow spheres, gold-coated silica nanoshells, and silica-coated gold shells. Preferred are gold nanoparticles.

In a further embodiment of the detection device of present inventions the first detector comprises an anti-IgM or anti-IgA antibody binding molecule conjugated to gold nanoparticles and an anti-IgG antibody binding molecule conjugated to carbon nanoparticles, and preferably an enzyme, more preferably horseradish peroxidase, or wherein the first detector comprises an anti-IgM or anti-IgA antibody binding molecule conjugated to carbon nanoparticles, and preferably an enzyme, more preferably horseradish peroxidase, and an anti-IgG antibody binding molecule conjugated to gold nanoparticles.

## BRIEF DESCRIPTION OF THE FIGURES

**Figure 1.** Illustrative drawing of the strip with its different zones (A), each of them overlapping 4 mm, as well as the cover and bottom of the housing of the testing device (B) and the strip located in the bottom of the housing. 1: Absorbent pad; 2: Membrane; 3: Conjugated pad; 4: Sample pad; 5: Sample collection window; 6: Conjugate window; 7: Result window; 8: Top of Housing; 9: Bottom of Housing; CL: Control line; TL: Test line

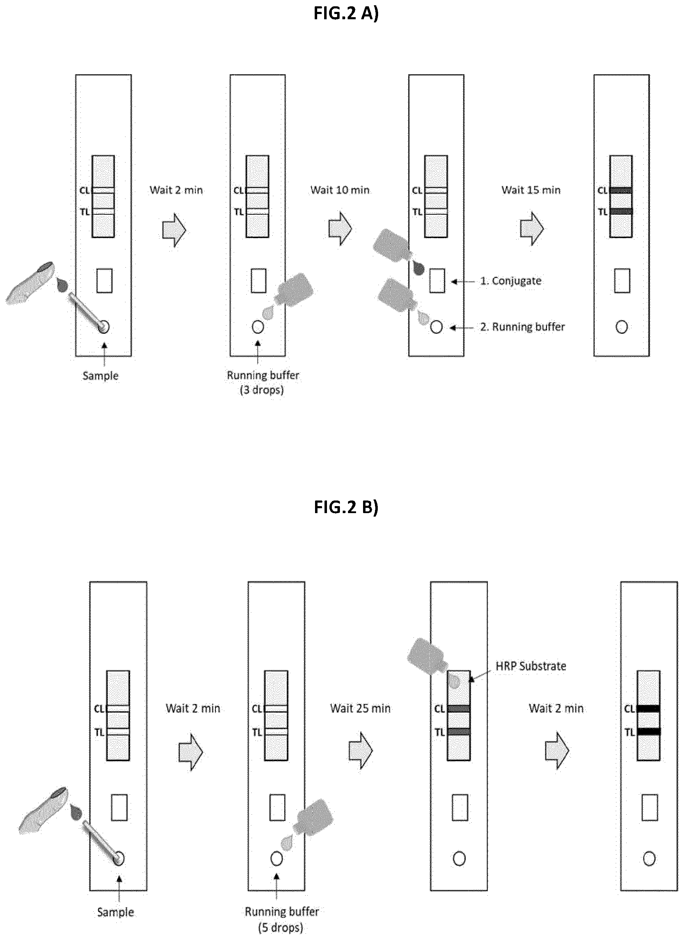

**Figure 2.** A) Illustrative drawing of one embodiment of the method of present invention adding a blood sample into the sample collection window, after 2 minutes adding running buffer into the sample collection window, after 10 minutes adding conjugate into the conjugate window and more running buffer into the sample collection window, after 15 minutes reading the results in the result window. B) Illustrative drawing of one embodiment of the method of present invention: Adding a blood sample into the sample collection window, after 2 minutes adding running buffer into the sample collection window, after 25 minutes adding Horseradish Peroxidase (HRP) Substrate onto the result window, after 2 minutes reading the results in the result window.

**Figure 3:** Illustrative drawing of possible results and their interpretation after performing the methods of present invention. Result is POSITIVE for target antibodies: both the test (TL) and the control line (CL) are black or grey coloured. Test line intensity correlated with the concentration of the antibodies in the sample. Darker test line indicates higher antibody concentration. Result is NEGATIVE for target antibodies: the test line (TL) does not develop colour, but the control line (CL) is coloured. Result is INVALID: There is no coloured control line (CL).

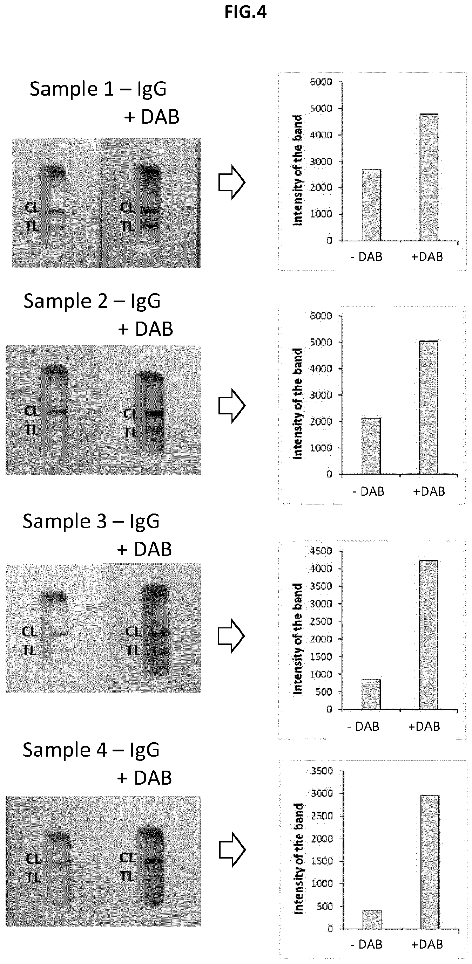

**Figure 4:** Photos of result window of exemplary test strips after performing the LFA for detection of IgG as per example 1 with and without addition of DAB (left side) and quantification of the intensity of the band (right side).

**Figure 5:** Photos of result window of exemplary test strips after performing the LFA for detection of IgA as per example 1 with and without addition of DAB (left side) and quantification of the intensity of the band (right side).

**Figure 6:** Photos of result window of exemplary test strips after performing the LFA for detection of IgM as per example 1 with and without addition of DAB (left side) and quantification of the intensity of the band (right side).

**Figure 7:** Illustrative non limited approach of the present invention for the simultaneous detection of COVID-19 IgG/IgM using two labels - carbon and gold nanoparticles - for combined signal generation.

**Figure 8:** Simultaneous signal detection of IgM and IgG using a strip with two test lines. Top: Photo showing the result window of LFAs performed according to Example 2, the test strip comprising two test lines, test line 1 for IgM and test line 2 for IgG detection. Bottom: Quantification of the band intensity.

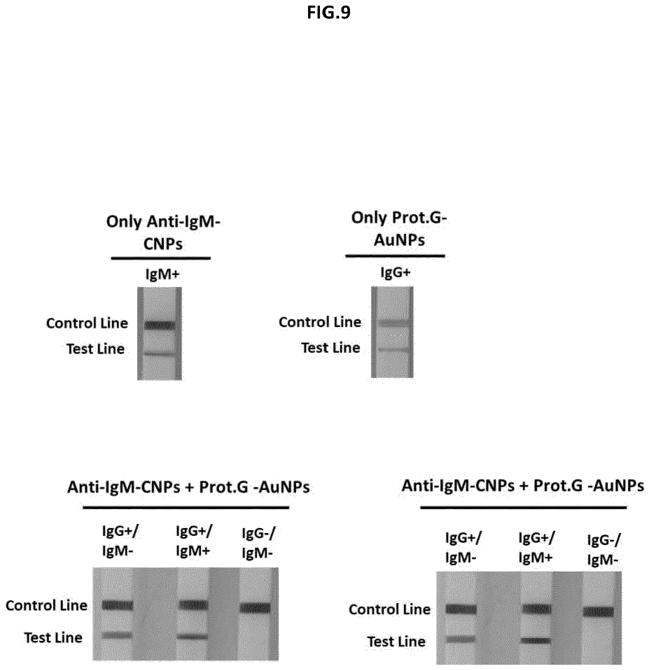

**Figure 9:** Simultaneous signal detection of IgM and IgG using a strip with one test line. Photos showing the result window of LFAs performed according to Example 2, the test strip comprising one test line. Top: Control showing detection of only IgM (left) or only IgG (right) visible via a black or red band, respectively. Bottom: Test showing simultaneous detection of both IgG and IgM in one test line visible via one red (IgG only present) and black band (IgM present, perhaps also with IgG). The photo on the right shows a higher intensity of the band, meaning that a larger amount of IgM was present in the sample than in the left photo.

**Figure 10:** Sensitivity of AuNPs and CNPs in LFAs. Photos of result window of LFAs performed with CNPs (upper strips) and AuNPs (lower strips) described in Example 3 using different dilutions without addition of DAB (top) and with addition of DAB (bottom).

**Figure 11:** Sensitivity of AuNPs and CNPs in LFAs. Quantification of the band intensity as shown in Fig.10.

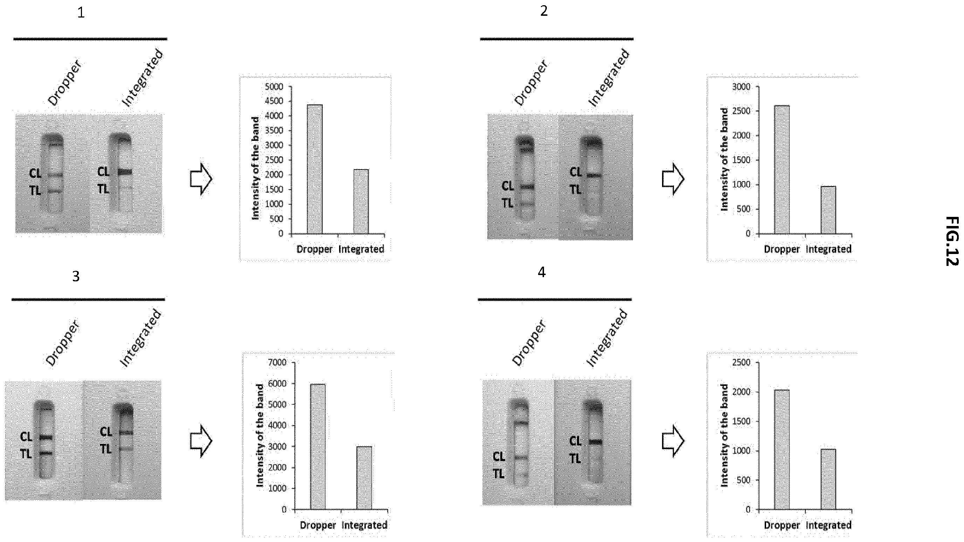

**Figure 12:** Photos of result window of exemplary test strips after performing the LFA for detection of IgG using the integrated and the dropper approach as per example 5 (left side) and quantification of the intensity of the bands (right side).

Figure 13. Illustrative drawing of the strip used in Examples 5 and 6. 1: Absorbent pad; 2: Membrane; 3: Conjugated pad; 4: Sample pad; CL: Control line; TL: Test line

## DESCRIPTION OF THE INVENTION

### Definitions

As used herein, the following terms shall have the following meanings:
The articles "a" and "an" are used herein to refer to one or to more than one (i.e., to at least one) of the grammatical Object of the article. By way of example, "an element" means one element or more than one element.

By "about" is meant a quantity, level, value, number, frequency, percentage, dimension, size, amount, weight or length that varies by as much as 30, 25, 20, 25, 10, 9, 8, 7, 6, 5, 4, 3, 2 or 1% to a reference quantity, level, value, number, frequency, percentage, dimension, size, amount, weight or length.

Throughout this specification, unless the context requires otherwise, the words "comprise," "comprises," and "comprising" will be understood to imply the inclusion of a stated step or element or group of steps or elements but not the exclusion of any other step or element or group of steps or elements.

By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements.

The terms "peptide" and "polypeptide" and "protein" are used interchangeably herein to refer to a polymer of amino acid residues and to variants and synthetic and naturally occurring analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers and naturally occurring chemical derivatives thereof.

The term "chromogenic substrate" is used to refer to substrates, usually peptides, that react with proteolytic enzymes, such as for example horseradish peroxidase, alkaline phosphatase or glucose oxidase, under the formation of color.

A "carbon nanoparticle" according to the present invention can be formed according to any suitable process capable of forming a carbon particle on a nanometre scale. For example, in one embodiment, a core carbon nanoparticle can be formed from an amorphous carbon source, such as carbon black; from graphite, for instance in the form of graphite powder; or from crystalline carbon (e.g., diamond). For example, according to one embodiment, a core carbon nanoparticle can be formed according to a laser ablation method from a graphite starting material. In another embodiment, a core carbon nanoparticle can be formed in an electric arc discharge from carbon powders. Other methods can be utilized as well, for instance, thermal carbonization of particles of carbon-rich polymers. Such methods are generally known to those of ordinary skill in the art and thus are not described in detail herein.

An "increased" or "enhanced" amount is optionally a "statistically significant" amount, and may include an increase that is about 1.1, 1.2, 1.3, 1.4, 1.5, 1.6, 1.7, 1.8, 1, 9, 2, 0, 2, 3, 4, 5, 6, 7, 8, 9, 10, 15, 20, 30 or more times (including all integers, ranges and decimal points in between and above 1, e.g., 2.5, 3.6, 3.7. 3.8, etc) the amount or value (e.g., signal or value such as a Reactivity score) relative, for example, to an antibody test performed without a detectable antibody binding molecule. A "increased" or "enhanced" value or amount may also include a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, 100%, 200%, 300%, 400%, 500% or more increase in the amount or value relative, for example, to an antibody test performed without a detectable antibody binding molecule.

All publications, patents and patent applications cited herein are hereby incorporated by reference in their entirety

### Detailed Description

There is an increasing demand for convenient and accurate point-of-care tools that can detect and diagnose diseases and ideally detect the different stages of the disease, even in remote or economically less affluent settings. In recent years, lateral flow Immunoassays (LFIA) have been indicated as a suitable medical diagnostic tool for these environments because they require little or no sample preparation, provide rapid and reliable results with no electronic components and thus can be manufactured at low costs and operated by unskilled personnel. However, even though they have been successfully applied to acute and chronic disease detection, LFIA show serious limitations when high sensitivity is needed. The inventors have therefore focused on improving the signal sensitivity of such assays to improve the possibilities of their application.

The signal generation approach of the present invention therefore relies on the use of carbon nanoparticles (CNPs), which significantly increase the sensitivity of the methods of present invention. The inventors have found that this approach can be potentiated even further by conjugating said CNPs with an enzyme, preferably a proteolytic enzyme that enhances the signal upon addition of a chromogenic substrate.

Therefore, one aspect of the present invention is directed to an *in vitro* method for detecting an antibody in a sample comprising the steps of

(a) contacting the sample with a first detector to form a complex comprising the first detector and the antibody, wherein the first detector comprises an antibody binding molecule conjugated to a first detectable entity, and wherein the first detectable entity comprises carbon nanoparticles, and preferably an enzyme;

(b) contacting the first complex with a capture entity immobilized on a test region of a surface, wherein the capture entity is capable of specifically binding to the antibody; and

(c) detecting the presence of a signal from the first detectable entity in the test region, wherein the presence of the signal is indicative of the presence of the antibody in the test sample.

In a further aspect the present invention provides for an *in vitro* method for detecting an antibody in a sample comprising the steps of:

(a) contacting the sample with a capture entity immobilized on a test region of a surface, wherein the capture entity is capable of specifically binding to the antibody to form a complex comprising the capture entity and the antibody;

(b) contacting the complex comprising the capture entity and the antibody with a first detector to form a complex comprising the first detector and the antibody, wherein the first detector comprises an antibody binding molecule conjugated to a first detectable entity, and wherein the first detectable entity comprises at least carbon nanoparticles, and preferably an enzyme; and

(c) detecting the presence of a signal from the first detectable entity in the test region, wherein the presence of the signal is indicative of the presence of the antibody in the test sample.

The signal generation approach of present invention is based on using a carbon core surface which is not photoluminescent due to the presence of, for example, a passivation agent coupled to said carbon core. Instead, the signal generation approach of the present invention is directed to develop a method in which the detectable signal is a colour change that can be identified with the naked eye thus making the use of expensive detectors unnecessary.

As described above, the signal generation approach of the present invention therefore relies on the use of CNPs. CNPs provide for a dark colour signal, which can be potentiated by an enzyme that significantly increases the intensity of the signal upon addition of its substrate.

In a preferred embodiment of the above-described methods, the enzyme used is a proteolytic enzyme selected from horseradish peroxidase, alkaline phosphatase and glucose oxidase, preferably the enzyme used is horseradish peroxidase (HRP).

In a further preferred embodiment, the horseradish peroxidase is conjugated to the CNPs.

The chromogenic substrate can be any chromogenic substrate known in the art, such as DAB (3,3'-diaminobenzidine), TMB (Tetramethylbenzidine), 2,2' -azino-di-[3-ethylbenzthiazoline-6-sulfonic acid] (ABTS), p-nitrophenylphosphate, p-aminophenylphosphate, BCIP/NBT (5-Bromo-4-chloro-3-indolyl phosphate along with nitro blue tetrazolium) or nuclear fast red. In one preferred embodiment the chromogenic substrate is DAB.

The reactants of the detection methods of present invention can be contacted in any order or sequence. For instance, the test sample can be mixed with the first detector to form a complex comprising the first detector and the antibody prior to application to the surface, or these two reactants can be applied separately to the surface, sequentially or at the same time, in the same or a different place on the surface. When added separately, the reactants will come into contact with one another as they spread or flow through the test surface, for example, by capillary or other action.

In particular embodiments, the first detector is immobilized to a conjugate region of the surface, wherein the conjugate region does not overlap with the test region of the surface.

In certain of these embodiments, the test sample can be applied to the surface, where it flows via capillary or other action through the conjugate region and the test region, and thereby contacts the complex comprising the first detector and the antibody and the capture entity that is capable of specifically binding to the antibody. If an antibody of interest is present in the sample, then it will form a complex with the first complex and the capture entity that is detectable as a signal in the test region, wherein the presence of the signal is indicative of the presence of the antibody in the test sample.

In certain embodiments, the conjugate region further comprises a control detector, for instance, an antibody that specifically binds to the antibody binding molecules. Other types of control regions will be apparent to persons skilled in the art and can be applied herein alternatively.

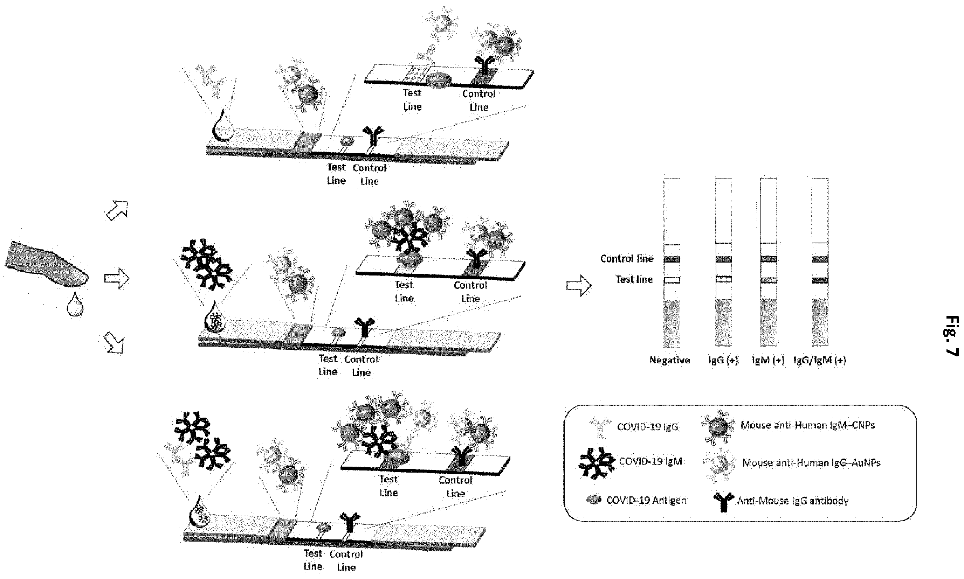

One approach performed by the inventors employed gold and carbon nanoparticles (see Fig.7). Specifically, anti-human IgG-AuNP and anti-human IgM-CNP conjugates were prepared. If the sample contains only IgG, the test lines will be coloured red, whilst if IgM (and possibly IgG) is present, the test line would be coloured black. Therefore, patients with a recent infection (IgM-containing samples) will be easily differentiated from patients with possible acquired immunity if no IgM is present (only IgG-containing samples). Moreover, the differentiation can be made with the naked eye, no detectors are necessary, thus providing for a cheap and simple detection.

An antibody, also referred to as an immunoglobulin, is a Y-shaped protein of the immune system that specifically identifies foreign objects or antigens, such as the components of bacteria, yeasts, parasites, and viruses. Each tip of the 'Y' of an antibody contains an antigen-binding site that is specific for a particular epitope on an antigen, allowing these two structures to bind together with precision. The production of a given antibody is increased upon exposure to an antigen (e.g., a microbial antigen) that specifically interacts with that antibody. Hence, the detection of antigen-specific antibodies in a sample from a subject can inform whether that subject is currently exposed to, or has been previously exposed to, a given microbe, such as a virus, bacteria, fungus, or parasite.

An "antigen-binding site," or "binding portion" of an antibody, refers to the part of the immunoglobulin molecule that participates in antigen binding. The antigen binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". In an antibody molecule, the three hypervariable regions of a light chain and the three hypervariable regions of a heavy chain are disposed relative to each other in three-dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs."

An antibody typically comprises all or a portion of an Fc region, to preferably facilitate detection by an Fc-binding molecule, and may also comprise one or more antigen-binding sites, to facilitate detection by an antibody-specific binding agent, such as an antigen or antigenic peptide. The antibodies can be, e.g., of IgG, IgE, IgD, IgM, or IgA type. Generally, IgM and/or IgA antibodies are detected for detection at early stages of infection, whereas later stages of an infection can be detected through IgG antibodies.

An antibody binding molecule according to the invention includes any binding agent that specifically binds to an antibody, such as to the Fc region or to any region that is outside of the variable region of an antibody. In certain embodiments, an antibody binding molecule is an Fc-specific secondary antibody, e.g., a rabbit anti-dog antibody, a goat anti-dog antibody. In certain embodiments, an antibody binding molecule is a secondary antibody against the antibody to be detected in a test sample. General examples of antibody binding molecules include polypeptides, soluble receptors, adnectins, small peptides, peptide mimetics, small molecules, aptamers, etc., that specifically bind to an immunoglobulin.

In one embodiment of the method of the present invention the antibody binding molecule is an anti-IgM or IgG or IgD or IgA or IgE, or a combination thereof, preferably an anti-IgM, anti-IgG or anti-IgA, or a combination thereof.

In one embodiment the antibody binding molecule is Protein G.

In a further embodiment the capture entity is an antigen or antigenic peptide, preferably a SARS-CoV-2 antigen or antigenic peptide.

In one embodiment of present invention said method comprises the simultaneous detection of IgM and IgG or IgA and IgG antibodies in the test sample.

In one preferred embodiment said method comprises the steps of

(a) contacting the test sample with a first detector to form a complex comprising the first detector and the antibody, wherein the first detector comprises an anti-IgM or anti-IgA antibody binding molecule conjugated to carbon nanoparticles, and preferably an enzyme, and an anti-IgG antibody binding molecule conjugated to metallic nanoparticle or metallic nanoshell, or wherein the first detector comprises an anti-IgG antibody binding molecule conjugated to carbon nanoparticles, and preferably an enzyme, and an anti-IgM or anti-IgA antibody binding molecule conjugated to metallic nanoparticle or metallic nanoshell;
(b) contacting the complex with a capture entity immobilized on a test region of a surface, wherein the capture entity is capable of specifically binding to the antibody; and
(c) detecting the presence of a signal from the combined signal generation after chromogenic substrate addition in the test region, wherein the presence of the signal is indicative of the presence of the antibody in the test sample and of the type of IgM and/or IgG and/or IgA antibody present in the test sample.

In one embodiment of this preferred embodiment the metallic nanoparticle or metallic nanoshell is selected from the group consisting of gold nanoparticles, silver particles, copper particles, platinum particles, cadmium particles, composite particles, gold hollow spheres, gold-coated silica nanoshells, and silica-coated gold shells. Preferred are gold nanoparticles.

In another preferred embodiment said method comprises the steps of

(a) contacting the test sample with a first detector to form a complex comprising the first detector and the antibody, wherein the first detector comprises an anti-IgM or anti-IgA antibody binding molecule conjugated to carbon nanoparticles, and preferably an enzyme, more preferably horseradish peroxidase, and an anti-IgG antibody binding molecule conjugated to a gold nanoparticle, or wherein the first detector comprises an anti-IgG antibody binding molecule conjugated to carbon nanoparticles, and preferably an enzyme, more preferably horseradish peroxidase, and an anti-IgM or anti-IgA antibody binding molecule conjugated to a gold nanoparticle;
(b) contacting the complex with a capture entity immobilized on a test region of a surface, wherein the capture entity is capable of specifically binding to the antibody; and
(c) detecting the presence of a signal from the combined signal generation after chromogenic substrate addition in the test region, wherein the presence of the signal is indicative of the presence of the antibody in the test sample and of the type of IgM or IgA and/or IgG antibody present in the test sample.

In one embodiment the enzyme is a proteolytic enzyme selected from horseradish peroxidase, alkaline phosphatase and glucose oxidase, preferably the enzyme used is horseradish peroxidase (HRP).

In one approach and in line with this embodiment, the authors of the present invention used two nanoparticle labels, gold nanoparticles (AuNPs) and carbon nanoparticles (CNPs) to simultaneously detect the presence of IgG and IgM antibodies in a sample as illustrated in Figure 7and further detailed in Example 2. The AuNPs, producing a red wine colour on the white nitrocellulose membrane, was conjugated to a human IgG antibody binding molecule for the detection of COVID-19 IgG antibodies, such as protein G or anti-IgG. On the other hand, the CNPs give a carbon-black colour and they were conjugated to a mouse anti-human IgM antibody for the detection of IgM antibodies. As shown in the exemplary Fig.7, if only IgG is present in the blood specimen, it will bind to the anti-human IgG-AuNPs conjugate adsorbed on the conjugate pad and captured on the testing zone by the COVID-19 antigen, resulting in a red-coloured line. When only IgM was present, it will bind to the anti-human IgM-CNPs conjugate and be captured on the test line where a black-coloured line will be formed.

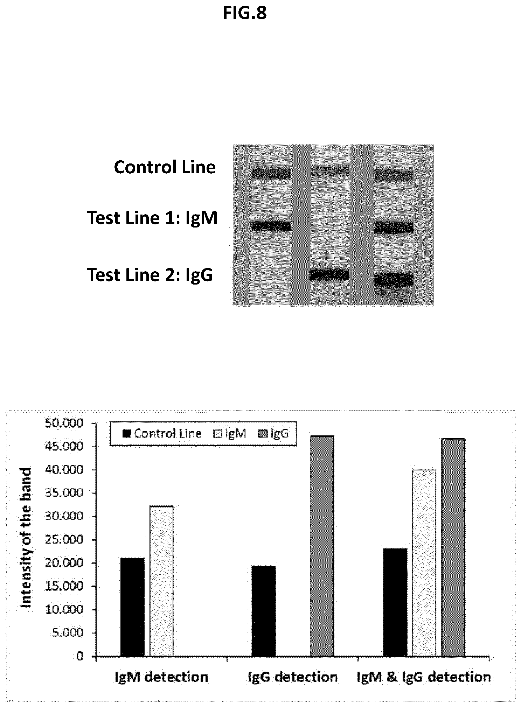

The inventors have performed this assay on test strips comprising in one case only one test line, and in the other case two test lines. The results for an LFA strip using two different test lines are shown in Fig.8. When only IgM was present in the sample, a band appeared in test line 1, whereas in a sample, where only IgG was present, a band only appeared in test line 2. In a sample comprising both antibodies, both bands appeared.

The results of an LFA strip using only one test line for both antibodies are shown in Fig.9. When only IgG was present in the sample, a red band appeared in the test line, showing the presence of the AuNPs, whereas in a sample, where only IgM was present, a black band appeared in the test line, showing the presence of CNPs. In a sample comprising both antibodies, both bands appeared simultaneously showing that AuNPs and CNPs were present.

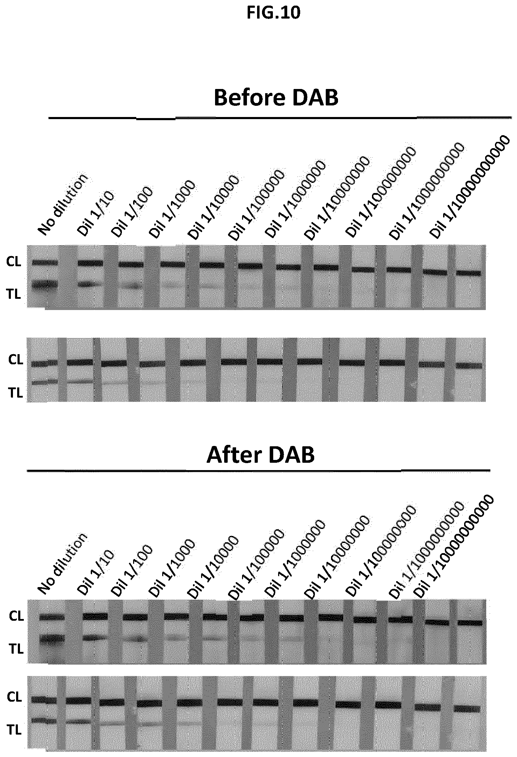

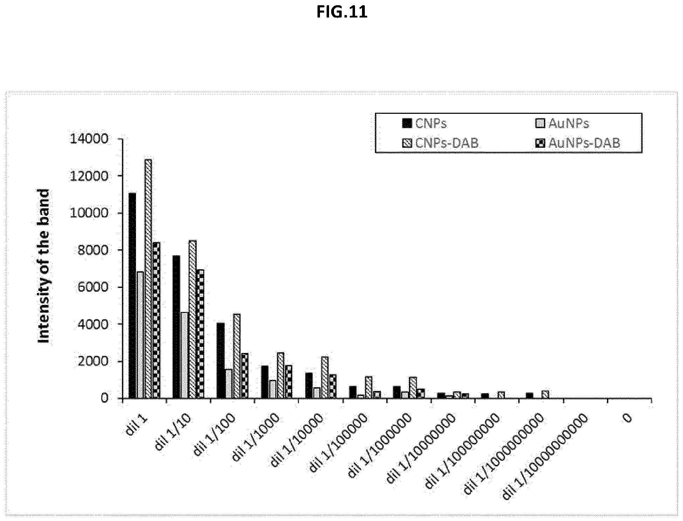

In a further approach the sensitivity of the test was assessed as described in Example 3. As can be seen in Figures 10 and 11 AuNP - IgG and CNP - IgM (with and w/o HRP) dilutions from 1/10 to $1/10^{10}$ were tested. The CNPs show a higher sensitivity and can be detected with the naked eye up to a dilution of $1/10^9$, whereas the AuNPs can still be detected up to a dilution of $1/10^7$. In both cases the addition of DAB increases the signal. It was found that addition of DAB is especially useful for intensity increase of the signal in lower concentrations. As such, the use of DAB can be omitted if a clear line can be seen within 15 minutes. However, if the line is not there or only very faint so that the result is not clearly readable with the naked eye, the addition of DAB can help to verify the result nevertheless, thus making it possible to even detect very small amount of antibody in the sample.

In one embodiment of the methods of present invention the said conjugate region further comprises a control detector.

In a further embodiment of present invention, the surface is a flow path in a lateral flow assay device.

In one embodiment of the present invention the test sample is a bodily fluid, extract of a bodily organ, blood, serum, or plasma.

According to present invention a test sample is a biological sample obtained from a subject that is suspected of having an antibody of interest, such as an antibody that is specific for an infectious agent or disease. The biological sample is preferably easy to obtain and may include blood, serum or plasma derived from a venous blood sample or even from a finger prick. Tissue from other body parts or other bodily fluids, such as cerebro-spinal fluid (CSF), saliva, gastric secretions, mucus, urine, faeces, etc., are known to contain antibodies and may be used as a source of a test sample. In other embodiments, the sample is a tissue (e.g., a tissue homogenate), extract from a bodily organ, or a cell lysate. In certain embodiments, the subject is a wild animal (e.g., a deer or rodent, such as a mouse, chipmunk, squirrel, etc.). In other embodiments, the subject is a lab animal (e.g., a mouse, rat, guinea pig, rabbit, monkey, primate, etc.). In other embodiments, the subject is a domesticated or feral animal (e.g., a dog, a cat, a horse). In still other embodiments, the subject is a human.

A capture entity according to present invention can be any binding agent that specifically binds to an antibody of interest, that is, a target antibody to be detected by the methods and devices described herein. Typically, the capture entity specifically binds to the variable region of an antibody, and thus contains one or more epitopes that are specific for the antigen-binding site(s) of an antibody. In certain embodiments, a capture entity is not an antibody or an antigen-binding fragment thereof. In particular embodiments, a capture entity is an antigen or an antigenic peptide that specifically binds to an antibody of interest. Exemplary antigens and antigenic peptides are described below. Also included are soluble receptors, adnectins, peptide mimetics, small molecules, aptamers, etc., that specifically bind to an antibody of interest, that is, an antibody that is to be detected according to the methods provided herein.

The capture entity of present invention is preferably attached to or immobilized on the test surface or substrate, such as a solid or semi-solid support. The attachment can be covalent or non-covalent, and can be facilitated by a moiety associated with the capture entity that enables covalent or non-covalent binding, such as a moiety that has a high affinity to a component attached to the carrier, support or surface. For example, the capture entity can be associated with a ligand, such as biotin, and the component associated with the surface can be a corresponding ligand receptor, such as avidin. Alternatively, a capture entity can be associated with a ligand receptor, such as avidin, and the component associated with the surface can be a corresponding ligand, such as biotin. The capture entity can be attached to or immobilized on the test surface or substrate either prior to or after the addition of a sample containing antibody during an immunoassay. In some embodiments of present invention, the test surface is a bead, dot blot, a flow path in a lateral flow assay device, or a flow path in an analytical rotor. For example, the capture entity can be attached or immobilized on a porous membrane, such as a PVDF membrane (e.g., an Immobilon™ membrane), a nitrocellulose membrane, polyethylene membrane, nylon membrane, or a similar type of membrane. In other embodiments, the test surface or substrate is a tube or a well, such as a well in a plate (e.g., a microtiter plate) suitable for use in an ELISA or other sandwich-type assay. In some embodiments, the test surface or substrate is a sensor, such as an electrochemical, optical, or opto-electronic sensor.

Such test surfaces or substrates can comprise glass, cellulose-based materials, thermoplastic polymers, such as polyethylene, polypropylene, or polyester, sintered structures composed of particulate materials (e.g., glass or various thermoplastic polymers), or cast membrane film composed of nitrocellulose, nylon, polysulphone, or the like. Suitable methods for immobilizing capture entities such as peptides on solid phases include ionic, hydrophobic, covalent interactions and the like. Specific or semi-specific binding to a solid or semi-solid carrier, support or surface, can be achieved by the capture entity having, associated with it, a moiety which enables its covalent or non-covalent binding to the solid or semi-solid carrier, support or surface. For example, the moiety can have affinity to a component attached to the carrier, support or surface. In this case, the moiety may be, e.g., a biotin or biotinyl group or an analogue thereof bound to an amino acid group of the peptide, such as 6-aminohexanoic acid, and the component is then avidin, streptavidin, neutravidin, or an analogue thereof.

As noted above, antigens and antigenic peptides can be used as capture entities and/or as antibody-specific detectors. A selected antigen or antigenic peptide is capable of specifically binding to a target antibody, most often via one or both of the antibody's antigen binding sites. Hence, the term "antigen," as used herein, refers to a molecule capable of being specifically bound by an antibody via at least one antigen-binding site of the antibody. An antigen can comprise one or more epitopes, the particular contiguous or non-contiguous region(s) of the antigen that specifically bind to the antigen-binding site of the antibody. An epitope can be a linear epitope, sequential epitope, or a conformational epitope. An antigen can be, for example, a peptide or a modified form thereof, or a non-peptide antigen such as a small molecule. As noted above, antigens can also include soluble receptors, adnectins, peptide mimetics, small molecules, aptamers, etc., that specifically bind to an antibody of interest. When used as capture entities, or "capture antigens," antigens or antigenic peptides are usually unlabelled and are immobilized or otherwise attached to a test surface. For certain embodiments, capture antigens can be fused or conjugated to or complexed with one or more heterologous proteins, such as bovine serum albumin or multiple antigen peptides (MAPS), to facilitate attachment to the test surface or other purpose.

Antigens and antigenic peptides and other antibody-specific binding agents can be derived from a variety of sources. Particular embodiments include those that are derived from microbial sources, including viruses, bacteria, fungi, and parasites. Specific examples include antigens that are from any one or more of heartworm, e.g., canine heartworm, Ehrlichia canis, Borrelia burgdorferi, Borrelia afzelii, Borrelia garinii, Anaplasma phagocytophilum, feline leukemia virus, parvovirus, e.g., canine parvovirus, influenza A strain, influenza B strain, avian influenza virus, respiratory syncytial virus, human immunodeficiency virus (HIV), Legionella, adenovirus, Group A Streptococcus, feline immunodeficiency virus (Fly), rotavirus, etc. Examples of Borrelia antigens for the detection of Lyme disease antibodies can be found in U.S. application Ser. No. 13/667,909, U.S. Patent Publication No. US 2011/0136155, and WO 2011/063003 (each of which is incorporated by reference in its entirety). Examples of Ehrlichia antigens for the detection of Ehrlichia antibodies can be found in U.S. Application No. 61/712,578, U.S. Patent Publication No. 2011/0124125, and WO2011/063235 (each of which is incorporated by reference in its entirety). Preferred antigens or antigenic peptides are derived from the SARS/COV2 virus and are useful for the detection of the IgM and IgG antibodies specifically produced by the human body's immune system to bind specific regions of the virus's structural proteins (S, E, M and N) as a response to the viral infection.

Antigenic peptides for use according to the methods described herein can be prepared by synthetic chemistry (i.e., a "synthetic peptide"). In other embodiments, antigenic peptides can be produced biologically (i.e., by cellular machinery, such as a ribosome). In certain embodiments, antigenic peptides are isolated. As used herein, an "isolated" peptide is a peptide that has been produced either synthetically or biologically and then purified, at least partially, from the chemicals and/or cellular machinery used to produce the peptide. In certain embodiments, an isolated peptide is substantially purified. The term "substantially purified," as used herein, refers to a molecule, such as a peptide, that is substantially free of cellular material (proteins, lipids, carbohydrates, nucleic acids, etc.), culture medium, chemical precursors, chemicals used in synthesis of the peptide, or combinations thereof. A peptide that is substantially purified has less than about 40%, 30%, 25%, 20%, 15%, 10%, 5%, 2%, 1% or less of the cellular material, culture medium, other polypeptides, chemical precursors, and/or chemicals used in synthesis of the peptide. Accordingly, a substantially pure molecule, such as a peptide, can be at least about 60%, 70%, 75%, 80%, 85%, 90%, 95%, 98%, or 99%, by dry weight, the molecule of interest. An isolated peptide can be in water, a buffer, or in a dry form awaiting reconstitution, e.g., as part of a kit. An isolated peptide can be in the form of a pharmaceutically acceptable salt. Suitable acids and bases that are capable of forming salts with the peptides of the present invention are well known to those of skill in the art and include inorganic and organic acids and bases.

An antibody of interest, or antigen-binding fragment thereof, is said to "specifically bind," "immunologically bind," and/or is "immunologically reactive" to a capture entity or antibody-specific detector (e.g., antigen or antigenic peptide) if it reacts at a detectable level (within, for example, a lateral flow assay, western blot, or ELISA assay) with the entity or detector, and does not react delectably in a statistically significant manner with unrelated polypeptides or agents under similar conditions. The term "specifically bind" can also mean that a capture entity or antibody-specific detector has a higher affinity (e.g., a higher degree of selectivity) for an antibody of interest than for other antibodies in a sample.

Immunological binding, as used in this context, generally refers to the non-covalent interactions of the type which occur between an immunoglobulin molecule and an antigen for which the immunoglobulin is specific. The strength, or affinity of binding such as immunological binding interactions can be expressed in terms of the dissociation constant ($K_d$) of the interaction, wherein a smaller $K_d$ represents a greater affinity. Immunological binding properties of selected polypeptides can be quantified using methods well known in the art (see, e.g., Davies et al., Annual Rev. Biochem. 59:439-473, 1990). In certain illustrative embodiments, an antibody has an affinity for a capture entity or an antibody-specific detector (e.g., antigen or antigenic peptide) of at least about 0.01, 0.05, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, or 50 nM. As another example, a capture entity or antibody-specific detector can have an affinity for the antibody of at least about 1.5-fold, 2-fold, 2.5-fold, 3-fold, or higher than for other antibodies in the sample. Similarly, antibody binding molecule is said to "specifically bind" to an immunoglobulin if it reacts at a detectable level with the Fc region and does not react detectably in a statistically significant manner with unrelated polypeptides or agents under similar conditions. In some illustrative embodiments, an antibody-binding molecule has an affinity for a selected immunoglobulin isotype of at least about 0.01, 0.05, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 26, 27, 28, 29, 30, 40, or 50 nM.

As noted above, the colorimetric antibody-binding molecules conjugated with CNPs are optionally connected or conjugated to HRP. Conjugation is typically achieved by covalent attachment.

Detecting the presence of a signal shall be achieved by the naked eye, that is, it shall permit the detection by visibly inspecting the capture complex for a colour change. A signal is typically indicative of the presence of an antibody of interest when it is stronger than the signal of negative control; however, not all tests require a negative control. In some instances, but not necessarily, the strength of a positive signal can be numerically quantified and is indicative of the presence of the antibody when that strength is statistically significant relative to a control. In some instances, the routine knowledge and experience with a particular test type establishes when a signal is positive or negative, and thus indicates the presence or absence of an antibody of interest. The detection signal in the present invention is significantly improved by the colorimetric antibody-binding molecules conjugated with CNPs being further connected or conjugated to HRP.

The protocols for immunoassays using antigens for detection of specific antibodies are well known in art. For example, a conventional sandwich assay can be used, or a conventional competitive assay format can be used. For a discussion of some suitable types of assays, see Current Protocols in Immunology (Coligan et al., editors, John Wiley & Sons, Inc). A particularly useful assay format is a lateral flow immunoassay format. As one non-limiting example, reporter or detector molecules including colorimetric antibody-binding molecules conjugated with CNPs and optionally connected or conjugated to HRP and optionally an antibody specific-antigen are dried and placed on a glass fibre pad (the sample application pad or conjugate pad). Unlabelled antigen or antigenic peptide (i.e., the capture entity) is immobilized on a membrane, such as nitrocellulose or a PVDF (polyvinylidene fluoride) membrane (e.g., an Immobilon™

membrane). When a solution of sample (blood, serum, etc.) is applied to the sample application pad (or flows through the conjugate pad), it dissolves the labelled detector(s), which then bind to the antibodies in the sample, if present. The resulting complexes are then transported into the next membrane (PVDF or nitrocellulose containing the capture entity) by capillary action. If antibodies against the capture entity are present, then they complex with the detector(s) and the diagnostic capture entity on the membrane, thereby generating a signal (e.g., a band that can be seen or visualized). If HRP is used a HRP reagent is further used.

It should be understood by one of skill in the art that any number of conventional protein assay formats, particularly immunoassay formats, may be designed to utilize the various embodiments described herein. This invention is thus not limited by the selection of the particular assay format, and is believed to encompass assay formats that are known to those of skill in the art. Phrases such as "sample containing an antibody" or "detecting an antibody in a sample" are not meant to exclude samples or determinations (e.g., detection attempts) where no antibody is contained or detected. In a general sense, this invention involves assays to determine whether an antibody produced in response to infection by an infectious microbe is present in a sample, irrespective of whether or not it is detected. Conditions for reacting antigens/peptides and antibodies so that they react specifically are well-known to those of skill in the art. See, e.g., Current Protocols in Immunology (Coligan et al., editors, John Wiley & Sons, Inc).

Devices for performing specific binding assays, especially immunoassays, are known and can be readily adapted for use in the present methods. Solid phase assays, in general, are easier to perform than heterogeneous assay methods which require a separation step, such as precipitation, centrifugation, filtration, chromatography, or magnetism, because separation of reagents is faster and simpler. Solid-phase assay devices include microtiter plates, flow-through assay devices (e.g., lateral flow immunoassay devices), dipsticks, and immunocapillary or immunochromatographic immunoassay devices.

In another aspect, the present invention relates to devices for detecting an antibody in a test sample. The present invention therefore also relates to an antibody detection device comprising:

(a) a sample loading region;
(b) a conjugate region, wherein said conjugate region comprises a mobilizable first detector including an antibody binding molecule conjugated to a first detectable entity, wherein the first detectable entity comprises carbon nanoparticles, and preferably an enzyme; and
(c) a test region, wherein said test region comprises an immobilized capture entity capable of specifically binding to the antibody;

wherein the sample loading region, the conjugate region and the test region are configured so that in operation a liquid sample when loaded into the sample loading region, is in fluid communication with the conjugate region and the test region.

According to one embodiment of present invention, the conjugate region does not overlap with the test region. However, these two regions as well as the sample region are configured so that in operation a liquid sample when loaded into the sample loading region, is in fluid communication with the conjugate region and the test region. In a preferred embodiment the test sample flows through or communicates with the conjugate and test regions via capillary action.

In some embodiments of present invention, the device further comprises a control region in fluid communication with a liquid sample when it is loaded to the sample loading region. Preferably, the liquid sample flows through or communicates with the control region via capillary action. In certain embodiments, the control region comprises an immobilized binding partner capable of specifically binding a control detector.

In a further embodiment, the devices further comprise an absorbent pad positioned downstream of the test region.

In one embodiment the conjugate region is positioned upstream of the sample loading region. In some embodiments, the conjugate region is positioned downstream of the sample loading region. In some embodiments, the sample loading region comprises a blood separator material.

In a preferred embodiment of the detection device the antibody binding molecule is an anti-IgM or IgG or IgD or IgA or IgE, or a combination thereof, preferably an anti-IgM, anti-IgG or anti-IgA, or a combination thereof.

In a further embodiment the capture entity is an antigen or antigenic peptide, preferably a SARS-CoV-2 antigen or antigenic peptide.

In one embodiment of the detection device of present invention the first detector comprises an anti-IgM or IgA antibody binding molecule conjugated to carbon nanoparticles, and preferably an enzyme, and an anti-IgG antibody binding molecule conjugated to a metallic nanoparticle or metallic nanoshell, or the first detector comprises an anti-IgG antibody binding molecule conjugated to carbon nanoparticles, and preferably an enzyme, and an anti-IgM or anti-IgA antibody binding molecule conjugated to a metallic nanoparticle or metallic nanoshell.

In a preferred embodiment the metallic nanoparticle or metallic nanoshell is selected from the group consisting of gold nanoparticles, silver particles, copper particles, platinum particles, cadmium particles, composite particles, gold hollow spheres, gold-coated silica nanoshells, and silica-coated gold shells. Preferred are gold nanoparticles.

In certain embodiments, a lateral flow device is constructed using a sample/blood separation pad, nitrocellulose membrane, and an upper wick placed in a housing. The nitrocellulose membrane is striped with a test line or region comprising a capture entity, such as an unlabelled, antibody-specific antigen (e.g., a peptide antigen or non-peptide antigen), and optionally a control line or region containing an antibody that is reactive to an antibody binding molecule. A sample suspected of containing an antibody of interest can be pre-mixed with a detectable antibody binding molecule according to the invention, and then applied to the lateral flow assay device. Or, rather than pre-mixing, the sample and the antibody binding molecule can be applied at the same time or sequentially to the assay device, in any order. Alternatively, the nitrocellulose membrane is prepared as above and further striped with a conjugate line or region comprising a detectable antibody-binding molecule according to the invention, where the conjugate and test regions do not overlap. In some embodiments, a sample suspected of containing an antibody of interest can then be applied to the lateral flow assay device. In other embodiments, a sample suspected of containing an antibody of interest can be pre-mixed with a detectable antigen-conjugate, which specifically binds to the antibody (typically having the same binding specificity as the capture entity), and then applied to the lateral flow assay device. Similar to above, rather than pre-mixing, the sample and the detectable antigen-conjugate can be applied at the same time or sequentially to the assay device, in any order.

In some embodiments, a lateral flow device is constructed using a sample/blood separation pad, nitrocellulose membrane, and an upper wick placed in a housing. The nitrocellulose membrane is striped with a test line or region comprising a capture entity, such as an unlabelled, antibody-specific antigen (e.g., a peptide antigen or non-peptide antigen), optionally a control line or region containing an antibody that is reactive to an antibody-binding molecule, and a conjugate line or region comprising a detectable antigen-conjugate, which specifically binds to the antibody (typically having the same binding specificity as the capture entity). The conjugate and test regions usually do not overlap. A sample suspected of containing an antibody of interest can be pre-mixed with a detectable antibody-binding molecule-conjugate, and then applied to the lateral flow assay device. Also, rather than pre-mixing, the sample and the antibody-binding molecule can be applied at the same time or sequentially to the assay device, in any order. In one exemplary embodiment, two-port devices may also be used for sequential application of a test sample, conjugate and chase buffer. In certain lateral flow devices, the nitrocellulose membrane is striped with a test line or region comprising a capture entity, such as an unlabelled, antibody-specific antigen (e.g., a peptide antigen or non-peptide antigen), optionally a control line or region containing an antibody that is reactive to an antibody binding molecule (indicated below), and a conjugate line or region comprising a detectable antibody binding molecule and a detectable antigen-conjugate, which specifically binds to the antibody (typically having the same binding specificity as the capture entity). A sample suspected of containing an antibody of interest can then be applied to the lateral flow assay device.

The *in vitro* method and the detection device as described herein above can also be used to follow-up IgG antibody levels following vaccination. In one embodiment the nucleocapsid protein used at the test line is then replaced with the spike protein or parts of the spike protein (e.g. RBD, S1 or S2 domains).

In a further embodiment the *in vitro* method and the detection device as described herein above can also be modified for the detection of MERS, immobilising the MERS nucleocapsid protein at the test line and detecting IgG / IgA / IgM antibodies.

In a further embodiment the *in vitro* method and the detection device as described herein above can be used for the screening for coeliac disease, wherein a duplex test with two test lines, tissue transglutaminase at one test line for the detection of IgA anti-tissue transglutaminase antibodies, and deamidated gliadin at the other test line for the detection of IgG anti-gliadin antibodies, is used.

In a further embodiment the *in vitro* method and the detection device as described herein above can be used for the screening for rheumatoid arthritis. In this embodiment rheumatoid factor and anti-CCP IgG and IgA antibodies are detected.

In a further embodiment the *in vitro* method and the detection device as described herein above can be used for screening for autoimmune diseases such as for example Hashimoto's thyroiditis, Multiple Sclerosis and Diabetes mellitus Type 1.

In a further embodiment the *in vitro* method and the detection device as described herein above can be used for the detection of allergies against the identified allergens (celery, cereals containing gluten (such as barley and oats), crustaceans (such as prawns, crabs and lobsters), eggs, fish, lupin, milk, molluscs (such as mussels and oysters), mustard, peanuts, sesame, soybeans, where the specific proteins for each of the allergens are immobilized at the test line, and allergen-specific IgE antibodies are detected. In one embodiment various allergen proteins can be immobilized at various test lines in one assay.

**EXAMPLES**

**Example 1: Antibody LFA CoV-SARS 2 test protocol**

1. Buffer preparation

The following buffers were prepared:

a) Blocking buffer for the membrane
10 mM Carbonate-Bicarbonate buffer pH 9.6, 5% (w/v) Skim milk powder, 0.5% Empigen detergent. (E.g.: 50 mL: 2.5 g of Skim milk, 250 $\mu$L Empigen, 2.5 mL 200 mM Carbonate-Bicarbonate buffer pH 9.6 and up to 50 mL Milli Q water).

b) Buffers for Conjugate preparation

-    Dilution buffer: 5 mM Borate buffer pH 8.8
-    Washing and storage buffer: 5 mM Borate buffer pH 8.8, 1% BSA (w/v) and 10% Sucrose (w/v).
-    Conjugate pad pre-treatment: 5 mM Borate buffer pH 8.8, 1% BSA, 0.05% Tween.
-    Running buffer: PBS-tween 20, 1% BSA, 1% NaCl

2. Conjugate preparation:

The conjugate was prepared according to the following protocol:

1. Prepare 1% (w/v) of carbon nanoparticles in Milli Q water. Sonicate the particles for 5 min (E.g.: 10 mg carbon in 1 mL of Milli Q water).
2. Dilute the particles to 0.2% in 5 mM Borate buffer pH 8.8 and sonicate for 5 min.
3. Add 0.350 mg/mL of antibody in the 0.2% carbon nanoparticles. (e.g.: 350 $\mu$g of IgM-HRP in 1 mL of 0.2% carbon) and incubate overnight at room temperature and tilt rotation.
4. Add a final concentration of 1% BSA to the solution and incubate for 30 min at room temperature and tilt rotation. (e.g. Add 100 $\mu$L of 10% BSA (w/v) in 1 mL of conjugate).
5. Centrifuge the conjugate at 15000 rpm for 30 min. Remove the supernatant and add 1 mL of the washing buffer (5 mM Borate buffer pH 8.8, 1% BSA, 10% Sucrose) and resuspend the pellet.
6. Resuspend the pellet in the initial volume with the washing buffer and store at 4ºC until use. (E.g. If you prepared 1 mL of carbon nanoparticles, resuspend with 1 mL of storage buffer). The final concentration of the conjugate is 0.2%.

3. Conjugate pad pre-treatment:

The conjugate pad was pre-treated according to the following protocol:

1. Prepare the following solution: 5 mM Borate buffer pH 8.8, 1% BSA and 0.05% Tween.
2. Immerse the conjugate pad (Ahlström 8951) in this solution and take it out and let dry at least for 2h at 37ºC. It is possible to let dry overnight.
3. Dilute the conjugate with the washing / storage buffer as follow:

a. Prot. G-HRP-CNPs: 0.1% (dilution 1 in 2 from the prepared stock (0.2%))
b. IgM-HRP-CNPs: 0.05% (dilution 1 in 4 from the prepared stock (0.2%))
c. IgA-HRP-CNPs: 0.05% (dilution 1 in 4 from the prepared stock (0.2%))

4. Add 15 $\mu$L of conjugate mixture for strip (0.4 cm). To prepare larger conjugate pads, calculate the volume considering dispensing 15 $\mu$L every 0.4 cm. Let dry at 37ºC for at least 2 h.

4. Membrane preparation:

The membrane was pre-treated according to the following protocol:

1. Dispense 2 mg/mL of anti-goat IgG antibody in the control line and 2 mg/mL of NP in the test line, both diluted in PBS, using Whatman FF120HP membrane (Cytiva Life Sciences). Set the operational conditions in the ALFRD dispenser from ClaremontBio as:

    a. Power: 7.5 V
    b. Syringe: 4.78 mm
    c. Volume: 1mL
    d. Rate: 0.3 mL/min
    e. Delay: 0:05 mm:ss

2. Let dry for at least 2h at 37ºC.
3. Immerse the membrane in the blocking solution for 15 min, then dry for at least 2h at 37ºC.
4. Store at 4ºC until use.

5. Recommended Cleaning and Storage Procedure for the dispenser:

1. Following reagent dispensing, purge lines of remaining fluid.
2. Rinse several times with dl water.
3. Follow with several washes of 1-10% bleach (if desired).
4. Flush out bleach with dl water.
5. Purge out water and store reagent lines dry.
6. To avoid cross-contamination of reagents, it is recommended to designate one reagent dispense tip / tubing per reagent (cat # 07.811.01).

6. LFA strips preparation:

The materials needed to manufacture the strips are:

- Absorbent pad: CF7 with a width of 2.2 cm (Cytiva Life Sciences).
- Membrane: Immobilised Whatman FF120HP membrane with a width of 2.5 cm (Cytiva Life Sciences).
- Conjugate pad: pre-treated Ahlström grade 8951 and dried with the conjugate with a width of 0.8cm.
- Sample pad: Ahlström grade 1663 with a width of 3 cm.
- Backing pad: MIBA-050, from DCN DX
- Cassette: PC14, from TV plastics

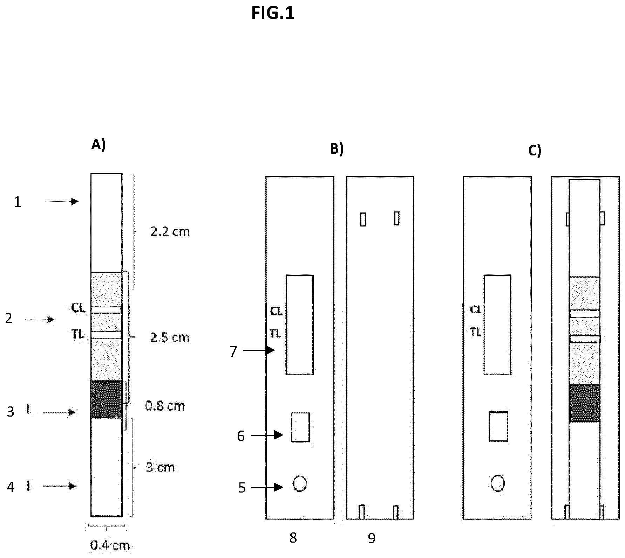

The strips were manufactured as shown in Fig.1, overlapping each pad by 4 mm.

7. Assay procedure with whole blood from finger-prick sample:

For performing the assay, the following protocol was performed with the subject to be tested (see Fig. 2A):

1. Clean the finger with ethanol and cotton.
2. Remove the security cap of the lancet, place the aperture of the lancet in close contact to the finger, press the actuator to pierce the outer layer of the finger and obtain the capillary blood sample. *Note: remove the first drop of blood with the cotton and press the finger to obtain fresh blood.*
3. Fill all the capillary with the blood (20 µL).
4. Add the sample to the sample collection window (small circle) in the strip by touching the end of the capillary to the membrane. Blood will flow by capillary force.

*Note: if blood does not flow, try to press the strip with less intensity.*

5. Wait 2 minutes to allow serum generation.
6. Add 5 drops of buffer in the same sample collection window (small circle).
7. Wait for a maximum of 30 minutes to read the result in the test window (big rectangle).
8. For the negative or faint positive results, add 1 drop of DAB reagent in the test window (big rectangle).

*Note: the DAB reagent should be prepared before use by adding 2700 µL of DAB buffer into the DAB bottle (which contains 300 µL of DAB).*

9. Wait for 2 minutes and read the result in the test window (big rectangle).

8. Assay procedure with plasma / serum sample:

    1. Add 10 μL of the sample to the sample collection window (small circle) in the strip.
    2. Add 5 drops of buffer in the same sample collection window (small circle).
    3. Wait for a maximum of 30 minutes to read the result in the test window (big rectangle).
    4. For these negative or faint positive results, add 1 drop of DAB reagent in the test window (big rectangle).

*Note: the DAB reagent should be prepared before use by adding 2700 μL of DAB buffer into the DAB bottle (which contains 300 μL of DAB).*
5. Wait for 2 minutes and read the result in the test window (big rectangle).

9. Result interpretation:

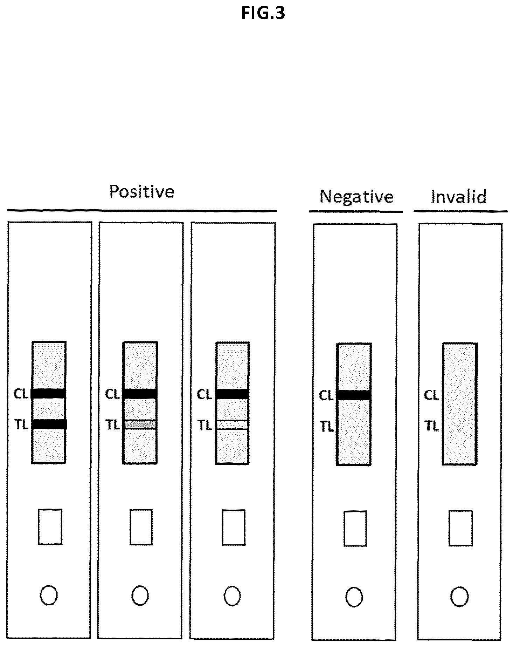

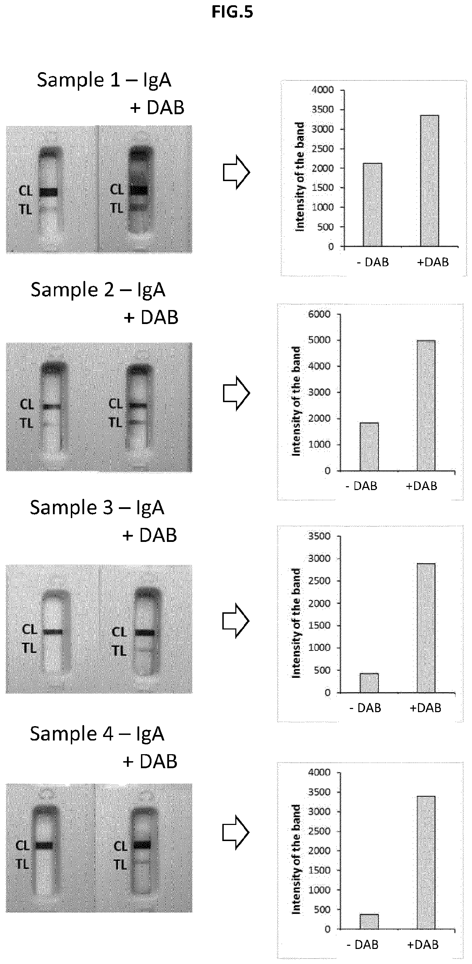

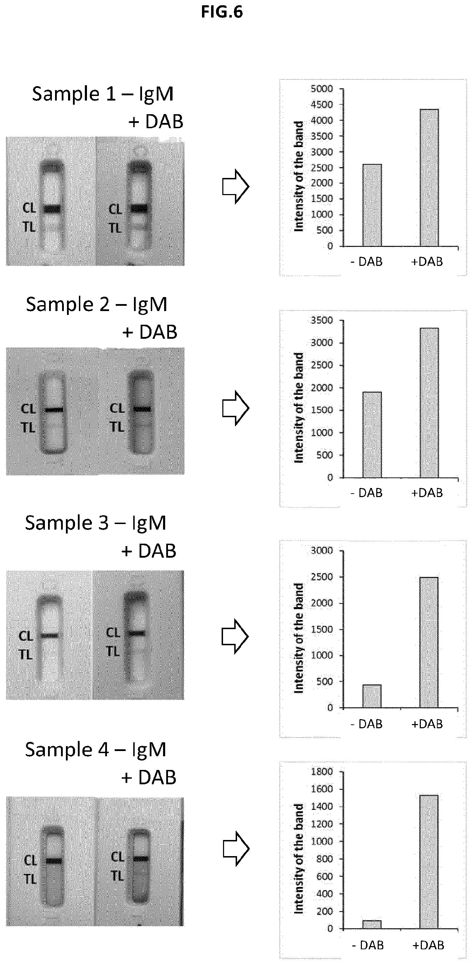

When the concentration of anti-SARS-CoV-2-NP antibodies in the sample is high, the positive results are visible within 10-15 minutes and the test can be completed. However, it is recommended to wait 20-30 minutes to read the results if a band is not observed at the test line within 15 minutes.
POSITIVE for IgG/IgM/IgA antibodies: both the test (TL) and the control line (CL) are black or grey coloured. Test line intensity correlated with the concentration of the antibodies in the sample. Darker test line indicates higher antibody concentration.
NEGATIVE for IgG/IgM/IgA antibodies: the test line (TL) does not develop colour, but the control line (CL) is coloured.
INVALID for IgG/IgM/IgA antibodies: There is no coloured control line (CL).
Examples of possible results can be seen in Fig.3.
The testing was performed with 100 samples of human sera from SARS-CoV-2 positive individuals as well as negative controls. Fig.5 - 7 show photos of exemplary test strips after performing the LFA as described above and showing a positive result for its respective targets, i.e. IgG, IgM and IgA.

**Example 2: Serologic LFA COV-SARS 2**

A serological test for the simultaneous detection of IgG and IgM antibodies was developed according to the following protocol.

1. Buffer preparation

- Blocking for the membrane: 10 mM Carbonate-Bicarbonate buffer pH 9.6, 5% (w/v) Skim milk powder, 0.5% Empigen detergent. (E.g.: 50 mL: 2.5 g of Skim milk, 250 μL Empigen, 2.5 mL 200 mM Carbonate-Bicarbonate buffer pH 9.6 and up to 50 mL Milli Q water).
- Conjugate preparation:

    ◦ Dilution buffer: 5 mM Borate buffer pH 8.8
    ◦ Washing and storage buffer: 5 mM Borate buffer pH 8.8, 1% BSA (w/v) and 10% Sucrose (w/v).

- Conjugate pad pre-treatment: 5 mM Borate buffer pH 8.8, 1% BSA, 0.05% Tween.
- Running buffer: PBS-tween 20, 1% BSA, 1% NaCl

2. Conjugate preparation:

CNPs- human anti-IgM antibody

    1. Prepare 1% (w/v) of carbon nanoparticles in Milli Q water. Sonicate the particles for 5 min (E.g.: 10 mg carbon in 1 mL of Milli Q water).

2. Dilute the particles to 0.2% in 5 mM Borate buffer pH 8.8 and sonicate for 5 min.

3. Add 0.350 mg/mL of antibody in the 0.2% carbon nanoparticles. (E.g.: 350 μg of human anti-IgM antibody in 1 mL of 0.2% carbon) and incubate overnight at room temperature and tilt rotation.

4. Add a final concentration of 1% BSA to the solution and incubate for 30 min at room temperature and tilt rotation. (E.g. Add 100 μL of 10% BSA (w/v) in 1 mL of conjugate).

5. Centrifuge the conjugate at 15000 rpm for 30 min. Remove the supernatant and add 1 mL of the washing buffer (5 mM Borate buffer pH 8.8, 1% BSA, 10% Sucrose) and resuspend the pellet.

6. Resuspend the pellet in the initial volume with the washing buffer and store at 4ºC until use. (E.g. If you prepared 1 mL of carbon nanoparticles, respuspend with 1 mL of storage buffer). The final concentration of the conjugate is 0.2%.

AuNPs- Protein G

1. Take directly the gold nanoparticles (AuNPs) from the bottle of BBI solutions with a size of 40 nm and OD 1 (BBI solutions: EM.GC40/8 Gold Colloid: 40nm -500ml). Sonicate the particles for 5 min.

2. Add 12.5 μg/mL of Protein G in the AuNPs OD 1. (E.g.: 12.5 μL of Protein G (1mg/mL) in 1 mL of AuNPs) and incubate for 30 min at room temperature and tilt rotation.

3. Add a final concentration of 1% BSA to the solution and incubate for 30 min at room temperature and tilt rotation. (E.g.: Add 100 μL of 10% BSA (w/v) in 1 mL of conjugate).

4. Centrifuge the conjugate at 15000 rpm for 30 min. Remove the supernatant and add 1 mL of the washing buffer (5 mM Borate buffer pH 8.8, 1% BSA, 10% Sucrose) and resuspend the pellet.

5. Resuspend the pellet 40x more concentrate with the washing buffer and store at 4ºC until use. (E.g.: If you prepared 1 mL of AuNPs, resuspend with 25 μL of storage buffer). The final concentration of the conjugate is OD 40.

3. Conjugate pad pre-treatment:

1. Prepare the following solution: 5 mM Borate buffer pH 8.8, 1% BSA and 0.05% Tween.

2. Immerse the conjugate pad (Ahlström 8951) in this solution and take it out and let dry at least for 2h at 37ºC. It is possible to let dry overnight.

3. Dilute the conjugate with the washing / storage buffer as follow:

   a. Human anti-IgM-CNPs: 0.1%
   b. Protein G-AuNPs: OD 30

4. Mix equally both conjugates (E.g.: 7 μL of human anti-IgM-CNPs 0.1% with 7 μL of Protein G-AuNPs OD 30). Add 15 μL of conjugate mixture for strip (0.4 cm). To prepare larger conjugate pad, calculate the volume considering to dispense 15 μL every 0.4 cm. Let dry at 37ºC for at least 2 h.

4. Membrane preparation:

1. Dispense 2 mg/mL of anti-goat IgG antibody in the control line and 2 mg/mL of NP in the test line, both diluted in PBS, using Whatman FF120HP membrane (Cytiva Life Sciences). Set the operational conditions in the ALFRD dispenser from ClaremontBio as:

   a. Power: 7.5 V
   b. Syringe: 4.78 mm
   c. Volume: 1 mL
   d. Rate: 0.3 mL/min
   e. Delay: 0:05 mm:ss

2. Let dry for at least 2h at 37ºC.

3. Immerse the membrane in the blocking solution for 15 min, then take out the membrane, and let dry for at least 2h at 37ºC.

4. Store at 4ºC until use.

5. LFA strips preparation:

The materials needed to manufacture the strips are:

- Absorbent pad: CF7 with a width of 2.2 cm (Cytiva Life Sciences).
- Membrane: Immobilised Whatman FF120HP membrane with a width of 2.5 cm (Cytiva Life Sciences).
- Conjugate pad: pre-treated Ahlström grade 8951 and dried with the conjugate with a width of 0.8cm.
- Sample pad: Ahlström grade 1663 with a width of 3 cm.
- Backing pad: MIBA-050, from DCN DX
- Cassette: PC14, from TV plastics

Manufacture the strips as it is shown in Fig.1, overlapping each pad 4 mm.

The strips were either prepared to contain two test lines, one for each antibody, or only one test line as shown in Fig.1, in which both antibodies can be detected in the same line.

The assay was performed as schematically depicted in Fig.8 (example with test strip with one test line) and following the protocol as described here below:

6. Assay procedure with whole blood from finger-prick sample:

1. Clean the finger with ethanol and cotton.

2. Remove the security cap of the lancet, place the aperture of the lancet in close contact to the finger, press the actuator to pierce the outer layer of the finger and obtain the capillary blood sample.

3. Fill all the capillary with the blood (20 µL).

4. Add the sample to the sample collection window (small circle) in the strip by touching the end of the capillary to the membrane. Blood will flow by capillary force.

5. Wait 2 minutes to allow serum generation.

6. Add 5 drops of buffer in the same sample collection window (small circle).

7. Wait for a maximum of 30 minutes to read the result in the test window (big rectangle).

7. Assay procedure with plasma / serum sample:

1. Add 10 µL of the sample to the sample collection window (small circle) in the strip.

2. Add 5 drops of buffer in the same sample collection window (small circle).

3. Wait for a maximum of 30 minutes to read the result in the test window (big rectangle).

8. Result interpretation:

When the concentration of anti-SARS-CoV-2-NP antibodies in the sample are high, the positive results are visible within 10-15 minutes. However, it is recommended to wait 20-30 minutes to read the results.

POSITIVE for IgG antibodies: both the test (TL) and the control line (CL) are coloured. The CL is brown coloured meanwhile the TL is red coloured. Test line intensity correlated with the concentration of the antibodies in the sample. Darker test line indicates higher antibody concentration.

POSITIVE for IgM antibodies: both the test (TL) and the control line (CL) are coloured. The CL is brown coloured meanwhile the TL is black or grey coloured. Test line intensity correlated with the concentration of the antibodies in the sample. Darker test line indicates higher antibody concentration.

POSITIVE for IgG and IgM antibodies: both the test (TL) and the control line (CL) are brown coloured. Test line intensity correlated with the concentration of the antibodies in the sample. Darker test line indicates higher antibody concentration.

NEGATIVE for IgG and IgM antibodies: the test line (TL) does not develop colour, but the control line (CL) is coloured.

INVALID for IgG and IgM antibodies: There is no coloured control line (CL).

As can be seen in Fig.9 and Fig.10 the use of multiple nanoparticles allows the simultaneous detection of multiple analytes, int his case IgM and IgG. In Fig.9, where the test strip comprises two test lines, one for IgM and one for IgG, it can be seen that when IgM only was detected a reddish band appeared in test line 1 whilst, when IgG only was detected, a blackish band appeared in test line 2. When both IgG and IgM are present both bands appeared.

Fig.10 shows the result after the test was performed with a test strip comprising only one test line in which both, IgG and IgM can be detected simultaneously. The same test line appears only in red if only IgG is present or in red and black if IgG and IgM are present. No signal is detected where none of the two are present.

## Example 3: Sensitivity of detection with AuNP and CNP

To assess the sensitivity of AuNP - IgG and CNP - IgM (with and w/o HRP) as prepared in Example 2, dilutions from 1/10 to $1/10^{10}$ were prepared and tested.

As can be seen in Figures 10 and 11, the CNPs show a higher sensitivity and can be detected with the naked eye up to a dilution of $1/10^9$, whereas the AuNPs can be detected up to a dilution of $1/10^7$. In both cases the addition of DAB increases the signal.

## Example 4: Test **validation of** test as **prepared in Example 1**

Using the 1000 serum samples from SARS-CoV-2 positive individuals, as well as negative samples obtained from pre-2019, the following specificity and sensitivities were obtained. We show the results with and without addition of the DAB substrate, as the addition of substrate does improve sensitivity, but affects specificity. The results were compared to results obtained using the ELISA used in Laboratori Clinic ICS Camp de Tarragona, Hospital Universitari de Tarragona Joan XXIII as well as with an in-house developed ELISA.

We define sensitivity and specificity as:

$$\text{Sensitivity} = (\text{True positives} / \text{True positives} + \text{false negatives})$$

$$\text{Specificity} = (\text{True negatives} / \text{True negatives} + \text{false positives})$$

Without addition of DAB substrate:

|     | SENSITIVITY | SPECIFICITY |
| --- | --- | --- |
| IgG | 90,3% | 100% |
| IgM | 97,4%. | 100% |
| IgA | 97,4% | 100% |

With addition of DAB substrate:

|     | SENSITIVITY | SPECIFICITY |
| --- | --- | --- |
| IgG | 100% | 93,3% |
| IgM | 99,8%. | 93,3% |
| IgA | 99,8% | 96,7% |

## Example 5: Serological LFA Serologic LFA COV-SARS 2 Integrated vs. Dropper approach

A serological test for the detection of IgG antibodies was developed according to the following protocol.

Buffer preparation

The following buffers were prepared:

Blocking for the membrane: 10 mM Carbonate-Bicarbonate buffer pH 9.6, 5% (w/v) Skim milk powder, 0.5% Empigen detergent. (E.g.: 50 mL: 2.5 g of Skim milk, 250 μL Empigen, 2.5 mL 200 mM Carbonate-Bicarbonate buffer pH 9.6 and up to 50 mL Milli Q water).

Conjugate preparation:

Dilution buffer: 5 mM Borate buffer pH 8.8

Washing and storage buffer: 5 mM Borate buffer pH 8.8, 1% BSA (w/v) and 10% Sucrose (w/v).

Conjugate pad pre-treatment: 5 mM Borate buffer pH 8.8, 1% BSA, 0.05% Tween.

Running buffer: PBS-tween 0.05% pH 7.4, 1% BSA, 1% NaCl

A) Dropper approach

1. Conjugate preparation:

1. Prepare 1% (w/v) of carbon nanoparticles in Milli Q water. Sonicate the particles for 5 min (E.g.: 10 mg carbon in 1 mL of Milli Q water).
2. Dilute the particles to 0.2% in 5 mM Borate buffer pH 8.8 and sonicate for 5 min.
3. Add 0.350 mg/mL of antibody or proteinG-HRP in the 0.2% carbon nanoparticles. (E.g.: 350 $\mu$g of antibody or Protein-G-HRP in 1 mL of 0.2% carbon) and incubate overnight at room temperature and tilt rotation.
4. Add a final concentration of 1% BSA to the solution and incubate for 30 min at room temperature and tilt rotation. (E.g. Add 100 $\mu$L of 10% BSA (w/v) in 1 mL of conjugate).
5. Centrifuge the conjugate at 15000 rpm for 30 min. Remove the supernatant and add 1 mL of the washing buffer (5 mM Borate buffer pH 8.8, 1% BSA, 10% Sucrose) and resuspend the pellet.
6. Resuspend the pellet in the initial volume with the washing buffer and store at 4ºC until use. (E.g. If you prepared 1 mL of carbon nanoparticles, resuspend with 1 mL of storage buffer).

2. Conjugate pad pre-treatment:

1. Prepare the following solution: 5 mM Borate buffer pH 8.8, 1% BSA and 0.05% Tween.
2. Immerse the conjugate pad (Ahlström 8951) in this solution and take it out and let dry at least for 2h at 37ºC. It is possible to let dry overnight.

3. Membrane preparation:

1. Dispense 2 mg/mL of Anti-Goat IgG antibody in the control line and 2 mg/mL Nucleoprotein in the test line diluted in PBS buffer pH 7.4 using Whatman FF80HP membrane (Cytiva Life Sciences) with the following conditions: 0.3 mL/min and 7.5 V (ClaremontBio dispenser).
2. Let the membrane dry for at least 2h at 37ºC.
3. Immerse the membrane in the blocking solution for 15 min, then take out the membrane and let dry for at least 2h at 37ºC.
4. Store at 4ºC until use.

4. LFA strips preparation:

The materials needed to manufacture the strips are:

- Absorbent pad: CF7 with a width of 2.2 cm (Cytiva Life Sciences).
- Membrane: Immobilised Whatman FF80HP membrane with a width of 2.5 cm (Cytiva Life Sciences).
- Conjugate pad: pre-treated Ahlström 8951 with a width of 2.3 cm.
- Sample pad: Cytosep 1663 with a width of 1.5 cm

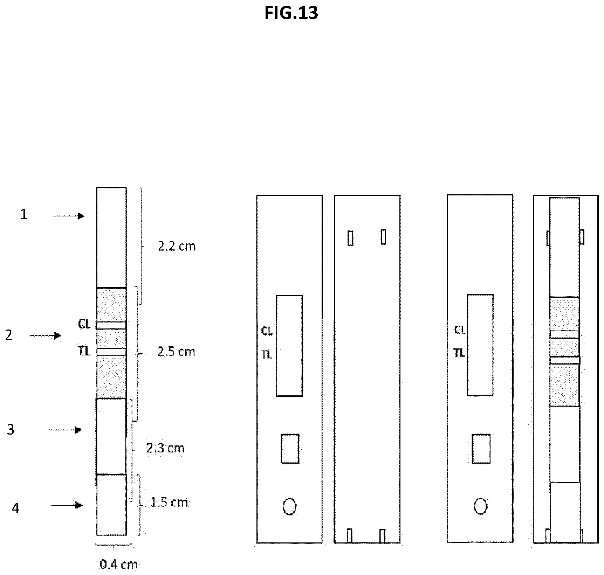

Manufacture the strips as shown in Fig.13, overlapping each pad 2 mm.

5. Perform assay as per desired option

LFA assay fingerprick:

1. Add 20 μL of blood with the capillary in the first inlet and wait 2 min for the serum generation.
2. Add 3 drops of running buffer in the first inlet (approx. 90 μL buffer). Wait 10 min.
3. Add 1 drop of conjugate (approx. 30 μL) in the second inlet (rectangular inlet). The conjugate has to be previous diluted to 0.05% for Prot.-G.-HRP, 0.025% for anti-IgM-HRP and Anti-IgA-HRP in the washing and storage buffer.
4. Add 1 drop of running buffer (approx. 30 μL) in the first inlet (circular inlet). Wait 15-20 min.

LFA assay Plasma / Serum:

1. Add 10 μL of plasma or serum in the first inlet (circular inlet).
2. Add 3 drops of running buffer in the first inlet (approx. 90 μL buffer). Wait 10 min.
3. Add 1 drop of conjugate (approx. 30 μL) in the second inlet (rectangular inlet). The conjugate has to be previous diluted to 0.05% for Prot.-G.-HRP, 0.025% for anti-IgM-HRP and Anti-IgA-HRP in the washing and storage buffer.
4. Add 1 drop of running buffer (approx. 30 μL) in the first inlet (circular inlet). Wait 15-20 min.

6. Result interpretation:

When the concentration of anti-SARS-CoV-2-NP antibodies in the sample are high, the positive results are visible within 10-15 minutes. However, it is recommended to wait 20-30 minutes to read the results.
POSITIVE for IgG/IgM/IgA antibodies: both the test (TL) and the control line (CL) are black or grey coloured. Test line intensity correlated with the concentration of the antibodies in the sample. Darker test line indicates higher antibody concentration.
NEGATIVE for IgG/IgM/IgA antibodies: the test line (TL) does not develop colour, but the control line (CL) is coloured.
INVALID for IgG/IgM/IgA antibodies: There is no coloured control line (CL).

B) Integrated approach

1. Conjugate preparation:

1. Prepare 1% (w/v) of carbon nanoparticles in Milli Q water. Sonicate the particles for 5 min (E.g.: 10 mg carbon in 1 mL of Milli Q water).
2. Dilute the particles to 0.2% in 5 mM Borate buffer pH 8.8 and sonicate for 5 min.
3. Add 0.350 mg/mL of antibody or proteinG-HRP in the 0.2% carbon nanoparticles. (E.g.: 350 μg of antibody or Protein-G-HRP in 1 mL of 0.2% carbon) and incubate overnight at room temperature and tilt rotation.
4. Add a final concentration of 1% BSA to the solution and incubate for 30 min at room temperature and tilt rotation. (E.g. Add 100 μL of 10% BSA (w/v) in 1 mL of conjugate).
5. Centrifuge the conjugate at 15000 rpm for 30 min. Remove the supernatant and add 1 mL of the washing buffer (5 mM Borate buffer pH 8.8, 1% BSA, 10% Sucrose) and resuspend the pellet. If you can resuspend the pellet well by pipetting or vortex, sonicate for 5 min and vortex again. Repeat this procedure at least 3 times.
6. Resuspend the pellet in the initial volume with the washing buffer and store at 4ºC until use. (E.g. If you prepared 1 mL of carbon nanoparticles, resuspend with 1 mL of storage buffer).

2. Conjugate pad pre-treatment:

1. Prepare the following solution: 5 mM Borate buffer pH 8.8, 1% BSA and 0.05% Tween.

2. Immerse the conjugate pad (Ahlström 8951) in this solution and take it out and let dry at least for 2h at 37ºC. It is possible to let dry overnight.

3. Dilute the conjugates with the washing / storage buffer as follow:

   a. Prot.G-HRP: 0.1%
   b. Anti-IgM-HRP: 0.05%
   c. Anti-IgA-HRP: 0.05%

4. Add 15 µL of conjugate for strip (0.4 cm). To prepare bigger conjugate pad, calculate the volume considering to dispense 15 µL every 0.4 cm. Let dry at 37ºC for at least 2 h.

### 3. Membrane preparation:

1. Dispense 2 mg/mL of Anti-Goat IgG antibody in the control line and 2 mg/mL Nucleoprotein in the test line diluted in PBS buffer pH 7.4 using Whatman FF120HP membrane (Cytiva Life Sciences) with the following conditions: 0.3 mL/min and 7.5 V (ClaremontBio dispenser).

2. Let the membrane dry for at least 2h at 37ºC.

3. Immerse the membrane in the blocking solution for 15 min, then take out the membrane and let dry for at least 2h at 37ºC.

4. Store at 4ºC until use.

### 4. LFA strips preparation:

The materials needed to manufacture the strips are:

- Absorbent pad: CF7 with a width of 2.2 cm (Cytiva Life Sciences).
- Membrane: Immobilised Whatman FF120HP membrane with a width of 2.5 cm (Cytiva Life Sciences).
- Conjugate pad: pre-treated Ahlström 8951 and dried with the desired conjugate with a width of 0.8 cm.
- Sample pad: Cytosep 1663 with a width of 3 cm.

Manufacture the strips as it is shown in Fig.1, overlapping each pad 2 mm.

### 5. Perform assay as per desired option

### LFA assay fingerprick:

1. Add 20 µL of blood with the capillary in the first inlet and wait 2 min for the serum generation.

2. Add 5 drops of running buffer in the first inlet (approx. 150 µL buffer). Wait 25 min. Read the test.

3. If it is needed, when low signal is observed in the test line, add 1 drop of HRP substrate directly on the membrane inlet. Wait 2 min and read the test.

### LFA assay Plasma / Serum:

1. Add 10 µL of plasma or serum in the first inlet (circular inlet).

2. Add 5 drops of running buffer in the first inlet (approx. 150 µL buffer). Wait 25 min. Read the test.

3. If it is needed, when low signal is observed in the test line, add 1 drop of HRP substrate directly on the membrane inlet. Wait 2 min and read the test.

### 6. Result interpretation:

When the concentration of anti-SARS-CoV-2-NP antibodies in the sample are high, the positive results are visible within 10-15 minutes. However, it is recommended to wait 20-30 minutes to read the results.

POSITIVE for IgG/IgM/IgA antibodies: both the test (TL) and the control line (CL) are black or grey coloured. Test line intensity correlated with the concentration of the antibodies in the sample. Darker test line indicates higher antibody concentration.

NEGATIVE for IgG/IgM/IgA antibodies: the test line (TL) does not develop colour, but the control line (CL) is coloured.

INVALID for IgG/IgM/IgA antibodies: There is no coloured control line (CL).

**Example 6: Serologic LFA for detection of food allergen specific IgE antibodies**

1. Buffer preparation

Blocking for the membrane: 10 mM Carbonate-Bicarbonate buffer pH 9.6, 5% (w/v) Skim milk powder, 0.5% Empigen detergent. (E.g.: 50 mL: 2.5 g of Skim milk, 250 $\mu$L Empigen, 2.5 mL 200 mM Carbonate-Bicarbonate buffer pH 9.6 and up to 50 mL Milli Q water).

Conjugate preparation:

Dilution buffer: 5 mM Borate buffer pH 8.8

Washing and storage buffer: 5 mM Borate buffer pH 8.8, 1% BSA (w/v) and 10% Sucrose (w/v).

Conjugate pad pre-treatment: 5 mM Borate buffer pH 8.8, 1% BSA, 0.05% Tween.

Running buffer: PBS-tween 0.05% pH 7.4, 1% BSA, 1% NaCl

A) Dropper approach

1. Conjugate preparation:

1. Prepare 1% (w/v) of carbon nanoparticles in Milli Q water. Sonicate the particles for 5 min (E.g.: 10 mg carbon in 1 mL of Milli Q water).
2. Dilute the particles to 0.2% in 5 mM Borate buffer pH 8.8 and sonicate for 5 min.
3. Add 0.350 mg/mL of anti-IgE-HRP to the 0.2% carbon nanoparticles. (E.g.: 350 $\mu$g of anti-IgE-HRP in 1 mL of 0.2% carbon) and incubate overnight at room temperature and tilt rotation.
4. Add a final concentration of 1% w/v BSA to the solution and incubate for 30 min at room temperature and tilt rotation. (E.g. Add 100 $\mu$L of 10% BSA (w/v) in 1 mL of conjugate).
5. Centrifuge the conjugate at 15000 rpm for 30 min. Remove the supernatant and add 1 mL of the washing buffer (5 mM Borate buffer pH 8.8, 1% BSA, 10% Sucrose) and resuspend the pellet. If you can resuspend the pellet well by pipetting or vortex, sonicate for 5 min and vortex again. Repeat this procedure at least 3 times.
6. Resuspend the pellet in the initial volume with the washing buffer and store at 4ºC until use. (E.g. If you prepared 1 mL of carbon nanoparticles, resuspend with 1 mL of storage buffer).

2. Conjugate pad pre-treatment:

1. Prepare the following solution: 5 mM Borate buffer pH 8.8, 1% BSA and 0.05% Tween.
2. Immerse the conjugate pad (Ahlström 8951) in this solution and take it out and let dry at least for 2h at 37ºC. It is possible to let dry overnight.

3. Membrane preparation:

1. Dispense 2 mg/mL of Anti-Goat IgG antibody on the control line and 2 mg/mL protein specific for the food allergen - examples of food allergens and examples of proteins that cause the food allergen are provided below. This list is not exclusive and other allergens could also be tested for, and other proteins could also be immobilised at the test line(s). Examples include celery (Api g 1), gluten (gliadin and glutenins), crustaceans and molluscs (tropomysin), eggs (ovomucoid and ovalbumin), fish (parvalbumin), lupin (beta -conglutin), milk (casein and whey), mustard (Sin A1, Sin S2, Sin A3, Sin A4), peanuts (Ara h1, Ara h 2, Ara h 3,iso Ara h3, Ara h 5, Ara h 6, Ara h 7, Ara h 8, Ara h 9, Ara h 10, Ara h 11, Ara h 12, Ara h 13, Ara h 14, Ara h 15, Ara h 16, Ara h 17), soybeans (soy protein)

2. The proteins are dispensed on the test line diluted in PBS buffer pH 7.4 using Whatman FF80HP membrane (Cytiva Life Sciences) with the following conditions: 0.3 mL /min and 7.5 V (ClaremontBio dispenser).

3. Let the membrane dry for at least 2h at 37ºC.

4. Immerse the membrane in the blocking solution for 15 min, then take out the membrane and let dry for at least 2h at 37ºC.

5. Store at 4ºC until use.

4. LFA strips preparation:

The materials needed to manufacture the strips are:

- Absorbent pad: CF7 with a width of 2.2 cm (Cytiva Life Sciences).
- Membrane: Immobilised Whatman FF80HP membrane with a width of 2.5 cm (Cytiva Life Sciences).
- Conjugate pad: pre-treated Ahlström 8951 with a width of 2.3 cm.
- Sample pad: Cytosep 1663 with a width of 1.5 cm

Manufacture the strips as shown in Fig.13, overlapping each pad 2 mm.

5. Perform assay as per desired option

LFA assay fingerprick:

1. Add 20 μL of blood with the capillary in the first inlet and wait 2 min for the serum generation.
2. Add 3 drops of running buffer in the first inlet (approx. 90 μL buffer). Wait 10 min.
3. Add 1 drop of conjugate (approx. 30 μL) in the second inlet (rectangular inlet). The conjugate has to be previous diluted to 0.025% in the washing and storage buffer.
4. Add 1 drop of running buffer (approx. 30 μL) in the first inlet (circular inlet). Wait 15-20 min.

LFA assay Plasma / Serum:

1. Add 10 μL of plasma or serum in the first inlet (circular inlet).
2. Add 3 drops of running buffer in the first inlet (approx. 90 μL buffer). Wait 10 min.
3. Add 1 drop of conjugate (approx. 30 μL) in the second inlet (rectangular inlet). The conjugate has to be previous diluted to 0.025% in the washing and storage buffer.
4. Add 1 drop of running buffer (approx. 30 μL) in the first inlet (circular inlet). Wait 15-20 min.

6. Result interpretation:

When the concentration of IgE antibodies in the sample are high, the positive results are visible within 10-15 minutes. However, it is recommended to wait 20-30 minutes to read the results.

POSITIVE for IgE: both the test (TL) and the control line (CL) are black or grey coloured. Test line intensity correlated with the concentration of the antibodies in the sample. Darker test line indicates higher antibody concentration.

NEGATIVE for IgE antibodies: the test line (TL) does not develop colour, but the control line (CL) is coloured.

INVALID for IgE antibodies: There is no coloured control line (CL).

B) Integrated approach

1. Conjugate preparation:

1. Prepare 1% (w/v) of carbon nanoparticles in Milli Q water. Sonicate the particles for 5 min (E.g.: 10 mg carbon in 1 mL of Milli Q water).
2. Dilute the particles to 0.2% in 5 mM Borate buffer pH 8.8 and sonicate for 5 min.
3. Add 0.350 mg/mL of anti-IgE HRP in the 0.2% carbon nanoparticles. (E.g.: 350 μg of anti-IgE HRP in 1 mL of 0.2% carbon) and incubate overnight at room temperature and tilt rotation.
4. Add a final concentration of 1% BSA to the solution and incubate for 30 min at room temperature and tilt rotation. (E.g. Add 100 μL of 10% BSA (w/v) in 1 mL of conjugate).
5. Centrifuge the conjugate at 15000 rpm for 30 min. Remove the supernatant and add 1 mL of the washing buffer (5 mM Borate buffer pH 8.8, 1% BSA, 10% Sucrose) and resuspend the pellet. If you can resuspend the pellet well by pipetting or vortex, sonicate for 5 min and vortex again. Repeat this procedure at least 3 times.
6. Resuspend the pellet in the initial volume with the washing buffer and store at 4ºC until use. (E.g. If you prepared 1 mL of carbon nanoparticles, resuspend with 1 mL of storage buffer).

2. Conjugate pad pre-treatment:

1. Prepare the following solution: 5 mM Borate buffer pH 8.8, 1% BSA and 0.05% Tween.
2. Immerse the conjugate pad (Ahlström 8951) in this solution and take it out and let dry at least for 2h at 37ºC. It is possible to let dry overnight.
3. Dilute the conjugate with the washing / storage buffer to 0.05% Anti-IgE-HRP
4. Add 15 μL of conjugate for strip (0.4 cm). To prepare bigger conjugate pad, calculate the volume considering to dispense 15 μL every 0.4 cm. Let dry at 37ºC for at least 2 h.

3. Membrane preparation:

1. Dispense 2 mg/mL of Anti-Goat IgG antibody on the control line and 2 mg/mL Nucleoprotein in the test line diluted in PBS buffer pH 7.4 using Whatman FF120HP membrane (Cytiva Life Sciences) with the following conditions: 0.3 mL/min and 7.5 V (ClaremontBio dispenser).
2. Let the membrane dry for at least 2h at 37ºC.
3. Immerse the membrane in the blocking solution for 15 min, then take out the membrane and let dry for at least 2h at 37ºC.
4. Store at 4ºC until use.

4. LFA strips preparation:

The materials needed to manufacture the strips are:

- Absorbent pad: CF7 with a width of 2.2 cm (Cytiva Life Sciences).
- Membrane: Immobilised Whatman FF120HP membrane with a width of 2.5 cm (Cytiva Life Sciences).
- Conjugate pad: pre-treated Ahlström 8951 and dried with the desired conjugate with a width of 0.8 cm.
- Sample pad: Cytosep 1663 with a width of 3 cm.

Manufacture the strips as shown in Fig.1, overlapping each pad 2 mm.

5. Perform assay as per desired option

LFA assay fingerprick:

1. Add 20 μL of blood with the capillary in the first inlet and wait 2 min for the serum generation.
2. Add 5 drops of running buffer in the first inlet (approx. 150 μL buffer). Wait 25 min. Read the test.
3. If it is needed, when low signal is observed in the test line, add 1 drop of HRP substrate directly on the membrane inlet. Wait 2 min and read the test.

LFA assay Plasma / Serum:

1. Add 10 μL of plasma or serum in the first inlet (circular inlet).
2. Add 5 drops of running buffer in the first inlet (approx. 150 μL buffer). Wait 25 min. Read the test.
3. If it is needed, when low signal is observed in the test line, add 1 drop of HRP substrate directly on the membrane inlet. Wait 2 min and read the test.

6. Result interpretation:

When the concentration of IgE antibodies in the sample are high, the positive results are visible within 10-15 minutes. However, it is recommended to wait 20-30 minutes to read the results.

POSITIVE for IgE antibodies: both the test (TL) and the control line (CL) are black or grey coloured. Test line intensity correlated with the concentration of the antibodies in the sample. Darker test line indicates higher antibody concentration.

NEGATIVE for IgE antibodies: the test line (TL) does not develop colour, but the control line (CL) is coloured.

INVALID for IgE antibodies: There is no coloured control line (CL).

**Claims**

**1.** An *in vitro* method for detecting an antibody in a test sample comprising:

(a) contacting the test sample with a first detector to form a complex comprising the first detector and the antibody, wherein the first detector comprises an antibody binding molecule conjugated to a first detectable entity, and wherein the first detectable entity comprises carbon nanoparticles and preferably an enzyme;
(b) contacting the first complex with a capture entity immobilized on a test region of a surface, wherein the capture entity is capable of specifically binding to the antibody; and
(c) detecting the presence of a signal from the first detectable entity in the test region, wherein the presence of the signal is indicative of the presence of the antibody in the test sample.

**2.** An *in vitro* method for detecting an antibody in a test sample comprising:

(a) contacting the test sample with a capture entity immobilized on a test region of a surface, wherein the capture entity is capable of specifically binding to the antibody to form a complex comprising the capture entity and the antibody;
(b) contacting the complex comprising the capture entity and the antibody with a first detector to form a complex comprising the first detector and the antibody, wherein the first detector comprises an antibody binding molecule conjugated to a first detectable entity, and wherein the first detectable entity comprises at least carbon nanoparticles, and preferably an enzyme; and
(c) detecting the presence of a signal from the first detectable entity in the test region, wherein the presence of the signal is indicative of the presence of the antibody in the test sample.

**3.** The method of claims 1 or 2, wherein the antibody is selected from IgM, IgG, IgD, IgA and IgE, or a combination thereof, preferably wherein the antibody is selected from IgM, IgG and IgA, or a combination thereof.

**4.** The method of claims 1 to 3, wherein the enzyme is selected from horseradish peroxidase, alkaline phosphatase, and glucose oxidase, preferably wherein the enzyme is horseradish peroxidase.

**5.** The method of claims 1 to 4, wherein the first detector is immobilized to a conjugate region of the surface and wherein the conjugate region does not overlap with the test region of the surface.

**6.** The method of any of claims 1 to 5, wherein said method comprises the simultaneous detection of IgM and IgG or IgA and IgG antibodies in the test sample.

**7.** The method of claim 6, wherein said method comprises

(a) contacting the test sample with a first detector to form a complex comprising the first detector and the antibody, wherein the first detector comprises an anti-IgM or anti-IgA antibody binding molecule conjugated to carbon nanoparticles and an anti-IgG antibody binding molecule conjugated to a metallic nanoparticle or metallic nanoshell, or wherein the first detector comprises an anti-IgG antibody binding molecule conjugated to carbon nanoparticles and an anti-IgM or anti-IgA antibody binding molecule conjugated to a metallic nanoparticle or metallic nanoshell;
(b) contacting the complex with a capture entity immobilized on a test region of a surface, wherein the capture entity is capable of specifically binding to the antibody; and
(c) detecting the presence of a signal from the combined signal generation after chromogenic substrate addition in the test region, wherein the presence of the signal is indicative of the presence of the antibody in the test sample and of the type of IgM, IgA and/or IgG antibody(ies) present in the test sample.

**8.** The method of claim 7, wherein the metallic nanoparticle or metallic nanoshell is selected from the group consisting of gold nanoparticles, silver particles, copper particles, platinum particles, cadmium particles, composite particles, gold hollow spheres, gold-coated silica nanoshells, and silica-coated gold shells, preferably wherein the metallic nanoparticle or metallic nanoshell are gold nanoparticles.

**9.** The method of claim 6, wherein said method comprises

(a) contacting the test sample with a first detector to form a complex comprising the first detector and the antibody, wherein the first detector comprises an anti-IgG antibody binding molecule conjugated to gold nanoparticles and an anti-IgM or anti-IgA antibody binding molecule conjugated to carbon nanoparticles, and preferably an enzyme, more preferably horseradish peroxidase; or wherein the first detector comprises an anti-IgM or anti-IgA antibody binding molecule conjugated to gold nanoparticles and an anti-IgG antibody binding molecule conjugated to carbon nanoparticles and preferably an enzyme, more preferably horseradish peroxidase;
(b) contacting the complex with a capture entity immobilized on a test region of a surface, wherein the capture entity is capable of specifically binding to the antibody; and
(c) detecting the presence of a signal from the combined signal generation after chromogenic substrate addition in the test region, wherein the presence of the signal is indicative of the presence of the antibody in the test sample and of the IgM, IgA and/or IgG antibody(ies) present in the test sample.

**10.** The method of any of claims 1 to 9, wherein the conjugate region further comprises a control detector and/or wherein the surface is a flow path in a lateral flow assay device.

**11.** The method of any of claims 1 to 10, wherein the test sample is a bodily fluid, extract of a bodily organ, blood, serum, or plasma.

**12.** An antibody detection device comprising:

a sample loading region;
a conjugate region, wherein said conjugate region comprises a mobilizable first detector including an antibody binding molecule conjugated to a first detectable entity, wherein the first detectable entity comprises carbon nanoparticles and preferably an enzyme; and
a test region, wherein said test region comprises an immobilized capture entity capable of specifically binding to the antibody;
wherein the sample loading region, the conjugate region and the test region are configured so that in operation a liquid sample when loaded into the sample loading region, is in fluid communication with the conjugate region and the test region.

**13.** The method of claims 1 to 11, or the detection device of claim 12, wherein the antibody binding molecule is an anti-IgM, anti-IgG, anti-IgD, anti-IgA or anti-IgE, or a combination thereof, preferably an anti-IgM, anti-IgG or anti-IgA, or a combination thereof.

**14.** The method of any of claims 1 to 11, or the detection device of any one of claims 12 or 13, wherein the capture entity is an antigen or antigenic peptide, preferably, wherein said antigen or antigenic peptide is a SARS-CoV-2 antigen or antigenic peptide.

**15.** The detection device of any of claims 12 to 14, wherein the first detector comprises an anti-IgM or anti-IgA antibody binding molecule conjugated to carbon nanoparticles, and preferably an enzyme, and an anti-IgG antibody binding molecule conjugated to a metallic nanoparticle or metallic nanoshell, or wherein the first detector comprises an anti-IgG antibody binding molecule conjugated to carbon nanoparticles, an preferably and enzyme, and an anti-IgM antibody binding molecule conjugated to a metallic nanoparticle or metallic nanoshell.

**FIG.1**

# FIG.2 A)

# FIG.2 B)

FIG.3

Positive     Negative   Invalid

## FIG.4

Sample 1 – IgG
+ DAB

Sample 2 – IgG
+ DAB

Sample 3 – IgG
+ DAB

Sample 4 – IgG
+ DAB

## FIG.5

FIG.6

Fig. 7

FIG.8

**FIG.9**

Only Anti-IgM-CNPs

IgM+

Control Line
Test Line

Only Prot.G-AuNPs

IgG+

Control Line
Test Line

Anti-IgM-CNPs + Prot.G -AuNPs

| IgG+/ IgM- | IgG+/ IgM+ | IgG-/ IgM- |

Control Line
Test Line

Anti-IgM-CNPs + Prot.G -AuNPs

| IgG+/ IgM- | IgG+/ IgM+ | IgG-/ IgM- |

Control Line
Test Line

FIG.10

**FIG.11**

# FIG.12

**FIG.13**

Europäisches
Patentamt

European
Patent Office

Office européen
des brevets

# EUROPEAN SEARCH REPORT

**Application Number**

EP 22 38 2087

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2013/130404 A1 (MEHRA RAJESH K [US] ET AL) 23 May 2013 (2013-05-23) * the whole document * * paragraphs [0003] – [0024], [0033] – [0044], [0057], [0058], [0062] – [0067], [0070] – [0077], [0079] – [0082], [0087] – [0111]; claims 1-21; figures 1-3 * | 1-15 | INV. G01N33/543 G01N33/558 G01N33/569 G01N33/58 G01N33/68 |
| Y | WO 2013/067524 A1 (ABAY SA [US]) 10 May 2013 (2013-05-10) * the whole document * * paragraphs [0007], [0008], [0012], [0013], [0017] – [0031], [0038], [0088] – [0100], [0105] – [0130]; claims 9-14 * | 1-15 | |
| Y | WO 2019/032669 A1 (ORASURE TECH INC [US]) 14 February 2019 (2019-02-14) * the whole document * * claims 1-27 * | 1-15 | |
| Y | ZHENGTU LI ET AL: JOURNAL OF MEDICAL VIROLOGY, vol. 92, no. 9, 13 April 2020 (2020-04-13) , pages 1518-1524, XP055737277, US ISSN: 0146-6615, DOI: 10.1002/jmv.25727 * the whole document * * abstract; figures 1,2; tables 1-3 * | 2-15 | TECHNICAL FIELDS SEARCHED (IPC) G01N |
| Y | WO 2017/066645 A1 (INBIOS INT INC [US]) 20 April 2017 (2017-04-20) * the whole document * | 1-15 | |
| Y | WO 2021/216276 A1 (MASSACHUSETTS INST TECHNOLOGY [US]) 28 October 2021 (2021-10-28) * the whole document * | 1-15 | |

-/--

The present search report has been drawn up for all claims

| Place of search | Date of completion of the search | Examiner |
|---|---|---|
| The Hague | 2 September 2022 | Boiangiu, Clara |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 1 of 2

42

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2020/208141 A1 (SPD SWISS PREC DIAGNOSTICS GMBH [CH]) 15 October 2020 (2020-10-15) * the whole document * ----- | 1-15 | |
| Y | WO 2021/224493 A1 (SENSEUTICS LTD [GB]) 11 November 2021 (2021-11-11) * the whole document * ----- | 1-15 | |
| Y | WO 2021/207209 A2 (UNIV COLUMBIA [US]) 14 October 2021 (2021-10-14) * the whole document * ----- | 1-15 | |
| Y | WO 2021/236640 A1 (UNIV MISSISSIPPI STATE [US]) 25 November 2021 (2021-11-25) * the whole document * ----- | 1-15 | |
| Y | US 8 956 859 B1 (BERMUDES DAVID G [US]) 17 February 2015 (2015-02-17) * the whole document * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 2 September 2022 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 220 166 A1

## ANNEX TO THE EUROPEAN SEARCH REPORT
## ON EUROPEAN PATENT APPLICATION NO.

EP 22 38 2087

02-09-2022

| Patent document cited in search report | | Publication date | Patent family member(s) | | Publication date |
|---|---|---|---|---|---|
| US 2013130404 | A1 | 23-05-2013 | AU | 2012340722 A1 | 22-05-2014 |
| | | | AU | 2018204793 A1 | 19-07-2018 |
| | | | CA | 2853812 A1 | 30-05-2013 |
| | | | CN | 104105965 A | 15-10-2014 |
| | | | CN | 106124757 A | 16-11-2016 |
| | | | CY | 1121291 T1 | 29-05-2020 |
| | | | DK | 2783216 T3 | 02-01-2019 |
| | | | EP | 2783216 A1 | 01-10-2014 |
| | | | EP | 3489685 A1 | 29-05-2019 |
| | | | ES | 2702033 T3 | 27-02-2019 |
| | | | HR | P20182023 T1 | 25-01-2019 |
| | | | HU | E041414 T2 | 28-05-2019 |
| | | | JP | 6267650 B2 | 24-01-2018 |
| | | | JP | 6742978 B2 | 19-08-2020 |
| | | | JP | 2015500459 A | 05-01-2015 |
| | | | JP | 2018072354 A | 10-05-2018 |
| | | | KR | 20140098750 A | 08-08-2014 |
| | | | KR | 20190049898 A | 09-05-2019 |
| | | | LT | 2783216 T | 25-01-2019 |
| | | | PL | 2783216 T3 | 29-03-2019 |
| | | | PT | 2783216 T | 19-12-2018 |
| | | | SI | 2783216 T1 | 31-01-2019 |
| | | | TR | 201818881 T4 | 21-01-2019 |
| | | | US | 2013130404 A1 | 23-05-2013 |
| | | | US | 2015293088 A1 | 15-10-2015 |
| | | | US | 2018156790 A1 | 07-06-2018 |
| | | | US | 2019219572 A1 | 18-07-2019 |
| | | | WO | 2013078227 A1 | 30-05-2013 |
| WO 2013067524 | A1 | 10-05-2013 | CA | 2851540 A1 | 10-05-2013 |
| | | | CN | 103998459 A | 20-08-2014 |
| | | | EP | 2773654 A1 | 10-09-2014 |
| | | | EP | 3502125 A1 | 26-06-2019 |
| | | | JP | 6839485 B2 | 10-03-2021 |
| | | | JP | 2015504420 A | 12-02-2015 |
| | | | JP | 2019048811 A | 28-03-2019 |
| | | | JP | 2021004251 A | 14-01-2021 |
| | | | KR | 20140091735 A | 22-07-2014 |
| | | | KR | 20190137172 A | 10-12-2019 |
| | | | US | 2013115634 A1 | 09-05-2013 |
| | | | US | 2014364332 A1 | 11-12-2014 |
| | | | US | 2016109446 A1 | 21-04-2016 |
| | | | US | 2017356910 A1 | 14-12-2017 |
| | | | US | 2018372743 A1 | 27-12-2018 |
| | | | US | 2021140964 A1 | 13-05-2021 |
| | | | WO | 2013067524 A1 | 10-05-2013 |

EPO FORM P0459

page 1 of 2

## ANNEX TO THE EUROPEAN SEARCH REPORT
## ON EUROPEAN PATENT APPLICATION NO.

EP 22 38 2087

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2022

| Patent document cited in search report | Publication date | Patent family member(s) | | Publication date |
|---|---|---|---|---|
| WO 2019032669 A1 | 14-02-2019 | CA | 3072436 A1 | 14-02-2019 |
| | | CN | 111164095 A | 15-05-2020 |
| | | EP | 3665187 A1 | 17-06-2020 |
| | | JP | 2020530567 A | 22-10-2020 |
| | | KR | 20200037846 A | 09-04-2020 |
| | | US | 2020371100 A1 | 26-11-2020 |
| | | WO | 2019032669 A1 | 14-02-2019 |
| WO 2017066645 A1 | 20-04-2017 | AU | 2016340125 A1 | 10-05-2018 |
| | | CA | 3001918 A1 | 20-04-2017 |
| | | EP | 3362180 A1 | 22-08-2018 |
| | | HK | 1257991 A1 | 01-11-2019 |
| | | KR | 20180088382 A | 03-08-2018 |
| | | US | 2017219573 A1 | 03-08-2017 |
| | | WO | 2017066645 A1 | 20-04-2017 |
| WO 2021216276 A1 | 28-10-2021 | NONE | | |
| WO 2020208141 A1 | 15-10-2020 | AU | 2020271401 A1 | 18-11-2021 |
| | | CA | 3136425 A1 | 15-10-2020 |
| | | CN | 113874729 A | 31-12-2021 |
| | | EP | 3953707 A1 | 16-02-2022 |
| | | US | 2022187325 A1 | 16-06-2022 |
| | | WO | 2020208141 A1 | 15-10-2020 |
| WO 2021224493 A1 | 11-11-2021 | NONE | | |
| WO 2021207209 A2 | 14-10-2021 | NONE | | |
| WO 2021236640 A1 | 25-11-2021 | NONE | | |
| US 8956859 B1 | 17-02-2015 | US | 8956859 B1 | 17-02-2015 |
| | | US | 9739773 B1 | 22-08-2017 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 667909
- US 20110136155 A
- WO 2011063003 A
- US 61712578
- US 20110124125 A
- WO 2011063235 A

**Non-patent literature cited in the description**

- **DAVIES et al.** *Annual Rev. Biochem.,* 1990, vol. 59, 439-473
- Current Protocols in Immunology. John Wiley & Sons, Inc